# EUROPEAN PATENT APPLICATION

(11) **EP 4 790 068 A1**
(43) Date of publication of application: **12.08.2026**
(21) Application number: 25157198.0
(22) Date of filing: 11.02.2025
(51) Int. Cl.: C07K 16/18, A61P 29/00

(54) **NEUTRALIZATION OF THE PRO-INFLAMMATORY EFFECT OF BACTERICIDAL/PERMEABILITY-IINCREASING PROTEIN (BPI)**

(71) Applicant: Universität Regensburg, in Vertretung des Freistaats Bayern, 93053 Regensburg (DE)
(72) Inventor: The designation of the inventor has not yet been filed
(74) Representative: Engelhard, Markus

(57) **Abstract**

The present invention relates to an antigen-binding peptide binding to bactericidal permeability-increasing protein (BPI). The present invention further relates to a nucleic acid encoding the antigen-binding peptide, a recombinant cell comprising the nucleic acid, and a composition comprising the antigen-binding peptide, the nucleic acid, or the recombinant cell. Furthermore, the present invention relates to the antigen-binding peptide, the nucleic acid, the recombinant cell, or the composition, for use in a method of preventing or treating an inflammatory disease, disorder, or condition. The present invention further relates to a use of the antigen-binding peptide, the nucleic acid, the recombinant cell, or the composition in a method of detecting BPI in a sample. The present invention further relates to a method of identifying an agent binding to BPI.

## Description

### FIELD OF THE INVENTION

The present invention relates to an antigen-binding peptide binding to bactericidal permeability-increasing protein (BPI). The present invention further relates to a nucleic acid encoding the antigen-binding peptide, a recombinant cell comprising the nucleic acid, and a composition comprising the antigen-binding peptide, the nucleic acid, or the recombinant cell. Furthermore, the present invention relates to the antigen-binding peptide, the nucleic acid, the recombinant cell, or the composition, for use in a method of preventing or treating an inflammatory disease, disorder, or condition. The present invention further relates to a use of the antigen-binding peptide, the nucleic acid, the recombinant cell, or the composition in a method of detecting BPI in a sample. The present invention further relates to a method of identifying an agent binding to BPI.

### BACKGROUND OF THE INVENTION

Bactericidal/permeability-increasing protein (BPI) is a cationic antimicrobial protein and is known for its bactericidal activity against Gram-negative bacteria as well as its anti-inflammatory capacities directed towards Gram-negative lipopolysaccharide. Despite this selectivity, BPI is elevated in infections of heterogeneous origin, including Gram-positive bacteria, fungi and viruses such as SARS-CoV-2. Consistent with enhanced neutrophil activation, increased BPI mRNA was additionally found in autoimmune disorders, including inflammatory bowel disease (IBD), and is associated with vascular pathology in systemic lupus erythematosus (SLE) and psoriasis. Moreover, elevation of BPI was found in affected joints of patients suffering from rheumatoid arthritis. BPI acts as a danger-associated molecular pattern towards dendritic cells, thereby inducing efficient activation of these cells.

Rheumatoid arthritis (RA) is a common autoimmune disease with a worldwide prevalence of 0.2 - 2% in the general population. In the course of disease, joints and multiple other organs are highly inflamed due to invasion of activated immune cells. Thereby, pro-inflammatory cytokines such as IL-6 and TNF cause a deleterious inflammation finally resulting in the destruction of affected joints. SLE is an autoimmune disease affecting approximately 0.1% of the population frequently affecting young women. Although SLE typically displays heterogenic clinical manifestations, increased cell death, abundant neutrophil activation and an increased type I interferon response finally leading to an uncontrolled activation of B cell are a hallmark of disease. SLE affects many organ systems, including skin, kidneys, brain, and the vasculature. Although multiple immunosuppressive approaches are established, severe long-term complication can occur such as premature cardiovascular disease or renal failure. The idiopathic inflammatory bowel disease (IBD) presents either as Crohn's disease or ulcerative colitis. In both entities an exaggerated immune response with lost tolerance against intestinal microbiota is found. Graft-versus-host disease (GVHD) is a common and often detrimental complication after allogeneic hematopoietic stem-cell transplantation, which is a potentially curative therapy for several hematologic disorders. Multiple immunosuppressive therapies are available for therapy of the diseases described above. Albeit, the treatment success is not achieved in a significant portion of the patients causing severe disabilities and even death. For inflammatory diseases, disorders, and conditions such as the diseases described above, effective therapies are urgently needed. Particularly, there is the need for means for anti-inflammatory treatments, such as means for inhibiting an immunostimulatory effect of BPI.

For example, there is a need for antigen-binding peptides, such as antibodies or antigen-binding fragments thereof, which neutralize BPI. Particularly, there is a need for antigen-binding peptides, such as antibodies or antigen-binding fragments thereof, which inhibit the immunostimulatory activity of BPI. Furthermore, there is the need for antigen-binding peptides, such as antibodies or antigen-binding fragments thereof, which inhibit the immunostimulatory activity of BPI and which do not inhibit the antibacterial activity of BPI. There is also the need for antigen-binding peptides, such as antibodies or antigen-binding fragments thereof, which bind to BPI with high affinity. There is also a need to enhance the prevention and treatment of inflammatory diseases, disorders, and conditions. Moreover, there remains the need for methods of detecting BPI in a sample. Additionally, there is the need for identifying further agents binding to BPI.

### SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to antigen-binding peptide binding to bactericidal permeability-increasing protein (BPI), wherein said antigen-binding peptide binds to an epitope of BPI comprising or consisting of an amino acid sequence selected from

| | |
|---|---|
| FPAAHDRMVYLGLSD | SEQ ID NO: 1; |
| VYLGLSDYFFNTAGLVYQE | SEQ ID NO: 2; |
| PVMEFPAAHDRMVYLGLSD | SEQ ID NO: 3; |
| TFLPEVAKKFPNM | SEQ ID NO: 4; |
| FQTLPVMTKIDSVAGINYLVAP | SEQ ID NO: 5; |
| HYSFYSMDIREFQLP | SEQ ID NO: 102; |
| MVPNVGLKFSISNANIKIS | SEQ ID NO: 103; and |
| QKRFLKMSGNFDLSI | SEQ ID NO: 104. |

In one embodiment, said antigen-binding peptide comprises any one of a)-n):
a) a heavy chain complementarity-determining region (HCDR) 1 comprising the amino acid sequence GFNFSTYG [SEQ ID NO: 6],
   a HCDR2 comprising the amino acid sequence ISGGGSYT [SEQ ID NO: 7], and
   a HCDR3 comprising the amino acid sequence ARHENGPWFAY [SEQ ID NO: 8]; or
b)
   a HCDR 1 comprising the amino acid sequence GFTFSIYD [SEQ ID NO: 9],
   a HCDR2 comprising the amino acid sequence VSGGGSYT [SEQ ID NO: 10],
   a HCDR3 comprising the amino acid sequence ARHENGPWFAY [SEQ ID NO: 11],
   a LCDR 1 comprising the amino acid sequence KSVSASDYSY [SEQ ID NO: 12],
   a LCDR2 comprising the amino acid sequence LAS [SEQ ID NO: 13], and
   a LCDR3 comprising the amino acid sequence QHSRELPYT [SEQ ID NO: 14]; or
c)
   a HCDR 1 comprising the amino acid sequence GFTFSTYG [SEQ ID NO: 15],
   a HCDR2 comprising the amino acid sequence VSGGGTYT [SEQ ID NO: 16],
   a HCDR3 comprising the amino acid sequence ARHETGPWFAY [SEQ ID NO: 17],
   a LCDR 1 comprising the amino acid sequence KSVSTSGYSY [SEQ ID NO: 18],
   a LCDR2 comprising the amino acid sequence LAS [SEQ ID NO: 19], and
   a LCDR3 comprising the amino acid sequence QHSRELPYT [SEQ ID NO: 20]; or
d)
   a HCDR 1 comprising the amino acid sequence GFSLISSA [SEQ ID NO: 21],
   a HCDR2 comprising the amino acid sequence IWAGGST [SEQ ID NO: 22],
   a HCDR3 comprising the amino acid sequence ATYYRFDDGFAY [SEQ ID NO: 23],
   a LCDR 1 comprising the amino acid sequence QSLVHSSGITY [SEQ ID NO: 24],
   a LCDR2 comprising the amino acid sequence RVS [SEQ ID NO: 25], and
   a LCDR3 comprising the amino acid sequence SQSTHFPYT [SEQ ID NO: 26]; or
e)
   a HCDR 1 comprising the amino acid sequence GFTFSNYG [SEQ ID NO: 27],
   a HCDR2 comprising the amino acid sequence ISGGGNYT [SEQ ID NO: 28],
   a HCDR3 comprising the amino acid sequence ARREAQLGSAMDY [SEQ ID NO: 29],
   a LCDR 1 comprising the amino acid sequence KSVSTSGYSY [SEQ ID NO: 30],
   a LCDR2 comprising the amino acid sequence LAS [SEQ ID NO: 31], and
   a LCDR3 comprising the amino acid sequence QHSRELPLT [SEQ ID NO: 32]; or
f)
   a HCDR 1 comprising the amino acid sequence GYEFSISW [SEQ ID NO: 33],
   a HCDR2 comprising the amino acid sequence IFPGDGDT [SEQ ID NO: 341,
   a HCDR3 comprising the amino acid sequence AREVGR [SEQ ID NO: 35],
   a LCDR 1 comprising the amino acid sequence QDIYNY [SEQ ID NO: 36],
   a LCDR2 comprising the amino acid sequence YTS [SEQ ID NO: 37], and
   a LCDR3 comprising the amino acid sequence QQGNTLPYT [SEQ ID NO: 38]; or
g)
   a LCDR 1 comprising the amino acid sequence QSIGTS [SEQ ID NO: 39],
   a LCDR2 comprising the amino acid sequence YAS [SEQ ID NO: 40], and
   a LCDR3 comprising the amino acid sequence QQSNSWPLT [SEQ ID NO: 41]; or
h)
   a HCDR 1 comprising the amino acid sequence GFDFDRYW [SEQ ID NO: 42],
   a HCDR2 comprising the amino acid sequence INPNNNTI [SEQ ID NO: 43],
   a HCDR3 comprising the amino acid sequence ARPGFYYGSAWFAY [SEQ ID NO: 44],
   a LCDR 1 comprising the amino acid sequence TGAVTTSNY [SEQ ID NO: 45],
   a LCDR2 comprising the amino acid sequence GSN [SEQ ID NO: 46], and
   a LCDR3 comprising the amino acid sequence ALWYNSHWV [SEQ ID NO: 47]; or
i)
   a HCDR 1 comprising the amino acid sequence GYTFTSYP [SEQ ID NO: 48],
   a HCDR2 comprising the amino acid sequence FHPYNDDT [SEQ ID NO: 49],
   a HCDR3 comprising the amino acid sequence ARAFYGNYDY [SEQ ID NO: 50],
   a LCDR 1 comprising the amino acid sequence QSVNND [SEQ ID NO: 51],
   a LCDR2 comprising the amino acid sequence YAS [SEQ ID NO: 52], and
   a LCDR3 comprising the amino acid sequence HQNNRSPWT [SEQ ID NO: 53]; or
j)
   a LCDR 1 comprising the amino acid sequence DHINNW [SEQ ID NO: 54],
   a LCDR2 comprising the amino acid sequence GTT [SEQ ID NO: 55], and
   a LCDR3 comprising the amino acid sequence QQYWSSPYT [SEQ ID NO: 56]; or
k)
   a HCDR 1 comprising the amino acid sequence GFTFSNYW [SEQ ID NO: 57],
   a HCDR2 comprising the amino acid sequence IRLKSNNYAT [SEQ ID NO: 58],
   a HCDR3 comprising the amino acid sequence TQSYYSMDY [SEQ ID NO: 59],
   a LCDR 1 comprising the amino acid sequence QSLLNSGNQKNY [SEQ ID NO: 60],
   a LCDR2 comprising the amino acid sequence GAS [SEQ ID NO: 61], and
   a LCDR3 comprising the amino acid sequence QNDHSYPRT [SEQ ID NO: 62]; or
l)
   a HCDR 1 comprising the amino acid sequence GFTFSNYW [SEQ ID NO: 63],
   a HCDR2 comprising the amino acid sequence IRLKSNNYAT [SEQ ID NO: 64],
   a HCDR3 comprising the amino acid sequence TQSYYSMDY [SEQ ID NO: 65],
   a LCDR 1 comprising the amino acid sequence QSLLYSSNQKNF [SEQ ID NO: 66],
   a LCDR2 comprising the amino acid sequence WAS [SEQ ID NO: 67], and
   a LCDR3 comprising the amino acid sequence QQYYNYPYT [SEQ ID NO: 68]; or
m)
   a HCDR 1 comprising the amino acid sequence GFTFSNYW [SEQ ID NO: 69],
   a HCDR2 comprising the amino acid sequence IRLRSNNYAS [SEQ ID NO: 70], and
   a HCDR3 comprising the amino acid sequence AQSYYAMDY [SEQ ID NO: 71]; or
n)
   a HCDR 1 comprising the amino acid sequence GFNIKDSY [SEQ ID NO: 72],
   a HCDR2 comprising the amino acid sequence IDPANGDT [SEQ ID NO: 73],
   a HCDR3 comprising the amino acid sequence ALYGYRAY [SEQ ID NO: 74],
   a LCDR 1 comprising the amino acid sequence QSLLNSGNQKNY [SEQ ID NO: 75],
   a LCDR2 comprising the amino acid sequence GAS [SEQ ID NO: 76], and
   a LCDR3 comprising the amino acid sequence QNDHSYPRT [SEQ ID NO: 77];

wherein, preferably, the antigen-binding peptide comprises any one of a)-j);
wherein, more preferably, the antigen-binding peptide comprises any one of b)-f), i) and h);
wherein, even more preferably, the antigen-binding peptide comprises any one of b)-f) and i).

In one embodiment, the antigen-binding peptide comprises a heavy chain variable sequence (VH) and a light chain variable sequence (VL), wherein VH and VL have at least 80%, preferably at least 90%, more preferably at least 95%, even more preferably at least 99%, optionally 100%, sequence identity to the amino acid sequences of:

| | | |
|---|---|---|
| i) | VH | SEQ ID NO: 78; or |
| ii) | VH | SEQ ID NO: 79 and |
| | VL | SEQ ID NO: 80, respectively; or |
| iii) | VH | SEQ ID NO: 81 and |
| | VL | SEQ ID NO: 82, respectively; or |
| iv) | VH | SEQ ID NO: 83 and |
| | VL | SEQ ID NO: 84, respectively; or |
| v) | VH | SEQ ID NO: 85 and |
| | VL | SEQ ID NO: 86, respectively; or |
| vi) | VH | SEQ ID NO: 87 and |
| | VL | SEQ ID NO: 88, respectively; or |
| vii) | VL | SEQ ID NO: 89, respectively; or |
| viii) | VH | SEQ ID NO: 90 and |
| | VL | SEQ ID NO: 91, respectively; or |
| ix) | VH | SEQ ID NO: 92 and |
| | VL | SEQ ID NO: 93, respectively; or |
| x) | VH | SEQ ID NO: 94, respectively; or |
| xi) | VH | SEQ ID NO: 95 and |
| | VL | SEQ ID NO: 96, respectively; or |
| xii) | VH | SEQ ID NO: 97 and |
| | VL | SEQ ID NO: 98, respectively; or |
| xiii) | VH | SEQ ID NO: 99; or |
| xiv) | VH | SEQ ID NO: 100 and |
| | VL | SEQ ID NO: 101, respectively |

wherein, preferably, the antigen-binding peptide comprises heavy and light chain variable sequences having at least 80%, preferably at least 90%, more preferably at least 95%, optionally identity, to the amino acid sequences of any one of i) - x);
wherein, more preferably, the antigen-binding peptide comprises heavy and light chain variable sequences having at least 80%, preferably at least 90%, more preferably at least 95%, optionally identity, to the amino acid sequences of any one of ii) - vi) and viii) - ix);
wherein, even more preferably, the antigen-binding peptide comprises heavy and light chain variable sequences having at least 80%, preferably at least 90%, more preferably at least 95%, optionally identity, to the amino acid sequences of any one of ii) - vi) and ix).

In one embodiment, said antigen-binding peptide binds to an epitope having SEQ ID NO: 1 and comprises
a)
   a heavy chain complementarity-determining region (HCDR) 1 comprising the amino acid sequence GFNFSTYG [SEQ ID NO: 6],
   a HCDR2 comprising the amino acid sequence ISGGGSYT [SEQ ID NO: 7], and
   a HCDR3 comprising the amino acid sequence ARHENGPWFAY [SEQ ID NO: 8]; or
b)
   a HCDR 1 comprising the amino acid sequence GFTFSIYD [SEQ ID NO: 9],
   a HCDR2 comprising the amino acid sequence VSGGGSYT [SEQ ID NO: 10],
   a HCDR3 comprising the amino acid sequence ARHENGPWFAY [SEQ ID NO: 11],
   a LCDR 1 comprising the amino acid sequence KSVSASDYSY [SEQ ID NO: 12],
   a LCDR2 comprising the amino acid sequence LAS [SEQ ID NO: 13], and
   a LCDR3 comprising the amino acid sequence QHSRELPYT [SEQ ID NO: 14]; or
c)
   a HCDR 1 comprising the amino acid sequence GFTFSTYG [SEQ ID NO: 15],
   a HCDR2 comprising the amino acid sequence VSGGGTYT [SEQ ID NO: 16],
   a HCDR3 comprising the amino acid sequence ARHETGPWFAY [SEQ ID NO: 17],
   a LCDR 1 comprising the amino acid sequence KSVSTSGYSY [SEQ ID NO: 18],
   a LCDR2 comprising the amino acid sequence LAS [SEQ ID NO: 19], and
   a LCDR3 comprising the amino acid sequence QHSRELPYT [SEQ ID NO: 20]; or
d)
   a HCDR 1 comprising the amino acid sequence GFSLISSA [SEQ ID NO: 21],
   a HCDR2 comprising the amino acid sequence IWAGGST [SEQ ID NO: 22],
   a HCDR3 comprising the amino acid sequence ATYYRFDDGFAY [SEQ ID NO: 23],
   a LCDR 1 comprising the amino acid sequence QSLVHSSGITY [SEQ ID NO: 24],
   a LCDR2 comprising the amino acid sequence RVS [SEQ ID NO: 25], and
   a LCDR3 comprising the amino acid sequence SQSTHFPYT [SEQ ID NO: 26]; or
e)
   a HCDR 1 comprising the amino acid sequence GFTFSNYG [SEQ ID NO: 27],
   a HCDR2 comprising the amino acid sequence ISGGGNYT [SEQ ID NO: 28],
   a HCDR3 comprising the amino acid sequence ARREAQLGSAMDY [SEQ ID NO: 29],
   a LCDR 1 comprising the amino acid sequence KSVSTSGYSY [SEQ ID NO: 30],
   a LCDR2 comprising the amino acid sequence LAS [SEQ ID NO: 31], and
   a LCDR3 comprising the amino acid sequence QHSRELPLT [SEQ ID NO: 32];
   or wherein said antigen-binding peptide binds to an epitope having SEQ ID NO: 2 and comprises
g)
   a LCDR 1 comprising the amino acid sequence QSIGTS [SEQ ID NO: 39],
   a LCDR2 comprising the amino acid sequence YAS [SEQ ID NO: 40], and
   a LCDR3 comprising the amino acid sequence QQSNSWPLT [SEQ ID NO: 41];
   or wherein said antigen-binding peptide binds to an epitope having SEQ ID NO: 3 and comprises
k)
   a HCDR 1 comprising the amino acid sequence GFTFSNYW [SEQ ID NO: 57],
   a HCDR2 comprising the amino acid sequence IRLKSNNYAT [SEQ ID NO: 58],
   a HCDR3 comprising the amino acid sequence TQSYYSMDY [SEQ ID NO: 59],
   a LCDR 1 comprising the amino acid sequence QSLLNSGNQKNY [SEQ ID NO: 60],
   a LCDR2 comprising the amino acid sequence GAS [SEQ ID NO: 61], and
   a LCDR3 comprising the amino acid sequence QNDHSYPRT [SEQ ID NO: 62]; or
l)
   a HCDR 1 comprising the amino acid sequence GFTFSNYW [SEQ ID NO: 63],
   a HCDR2 comprising the amino acid sequence IRLKSNNYAT [SEQ ID NO: 64],
   a HCDR3 comprising the amino acid sequence TQSYYSMDY [SEQ ID NO: 65],
   a LCDR 1 comprising the amino acid sequence QSLLYSSNQKNF [SEQ ID NO: 66],
   a LCDR2 comprising the amino acid sequence WAS [SEQ ID NO: 67], and
   a LCDR3 comprising the amino acid sequence QQYYNYPYT [SEQ ID NO: 68]; or
m)
   a HCDR 1 comprising the amino acid sequence GFTFSNYW [SEQ ID NO: 69],
   a HCDR2 comprising the amino acid sequence IRLRSNNYAS [SEQ ID NO: 70], and
   a HCDR3 comprising the amino acid sequence AQSYYAMDY [SEQ ID NO: 71];
   or wherein said antigen-binding peptide binds to an epitope having SEQ ID NO: 4 and comprises
f)
   a HCDR 1 comprising the amino acid sequence GYEFSISW [SEQ ID NO: 33],
   a HCDR2 comprising the amino acid sequence IFPGDGDT [SEQ ID NO: 341,
   a HCDR3 comprising the amino acid sequence AREVGR [SEQ ID NO: 35],
   a LCDR 1 comprising the amino acid sequence QDIYNY [SEQ ID NO: 36],
   a LCDR2 comprising the amino acid sequence YTS [SEQ ID NO: 37], and
   a LCDR3 comprising the amino acid sequence QQGNTLPYT [SEQ ID NO: 38];
   or wherein said antigen-binding peptide binds to an epitope having SEQ ID NO: 5 and comprises
n)
   a HCDR 1 comprising the amino acid sequence GFNIKDSY [SEQ ID NO: 72],
   a HCDR2 comprising the amino acid sequence IDPANGDT [SEQ ID NO: 73],
   a HCDR3 comprising the amino acid sequence ALYGYRAY [SEQ ID NO: 74],
   a LCDR 1 comprising the amino acid sequence QSLLNSGNQKNY [SEQ ID NO: 75],
   a LCDR2 comprising the amino acid sequence GAS [SEQ ID NO: 76], and
   a LCDR3 comprising the amino acid sequence QNDHSYPRT [SEQ ID NO: 77];
   or wherein said antigen-binding peptide binds to an epitope having any one of SEQ ID NO: 102-104 and comprises
i)
   a HCDR 1 comprising the amino acid sequence GYTFTSYP [SEQ ID NO: 48],
   a HCDR2 comprising the amino acid sequence FHPYNDDT [SEQ ID NO: 49],
   a HCDR3 comprising the amino acid sequence ARAFYGNYDY [SEQ ID NO: 50],
   a LCDR 1 comprising the amino acid sequence QSVNND [SEQ ID NO: 51],
   a LCDR2 comprising the amino acid sequence YAS [SEQ ID NO: 52], and
   a LCDR3 comprising the amino acid sequence HQNNRSPWT [SEQ ID NO: 53].

In one embodiment, said antigen-binding peptide is an antibody or an antigen-binding fragment thereof; wherein, preferably, said antigen-binding peptide is selected from an antibody, a Fab, Fab', a F(ab')2, a scFv, di-scFv, a V_{H} domain, a single-domain antibody (sdAb), a diabody, a triabody, and a tetrabody; wherein, more preferably, the antigen-binding peptide is selected from a humanized antibody, a chimeric antibody, and antigen-binding fragments thereof.

In one embodiment, said antigen-binding peptide is an IgG2a antibody, an IgG2b antibody, an IgG1 antibody, or an antigen-binding fragment of an IgG2a antibody, of an IgG2b antibody, or of an IgG1 antibody;
wherein, optionally, said antigen binding peptide is selected from a humanized IgG2a antibody, a humanized IgG2b antibody, a humanized IgG1 antibody, and antigen-binding fragments thereof.

In one embodiment, said antigen-binding peptide neutralizes said BPI; wherein, preferably, said antigen-binding peptide inhibits a pro-inflammatory activity of BPI in immune cells, particularly dendritic cells, leukocytes, and/or macrophages; wherein, preferably, said antigen-binding peptide inhibits an induction of cytokines, particularly IL-2 and/or TNF, by BPI in immune cells, particularly in dendritic cells, leukocytes, and/or macrophages, such as in bone marrow-derived dendritic cells.

In one embodiment, said antigen-binding peptide does not inhibit an antibiotic activity of BPI, particularly a bactericidal activity of BPI.

In one embodiment, said antigen-binding peptide has a KD of 10 nM or less, preferably has a KD of 7 nM or less, more preferably has a KD of 1.5 nM or less, even more preferably has a KD of 1.1 nM or less, still even more preferably has a KD of 0.5 nM or less, still even more preferably has a KD of 0.3 nM or less, such as a KD of 0.01 nM or less.

In a further aspect, the present invention relates to a nucleic acid, particularly an isolated nucleic acid, encoding an antigen-binding peptide as defined herein.

In a further aspect, the present invention relates to a recombinant cell comprising a nucleic acid as defined herein.

In a further aspect, the present invention relates to a composition, preferably a pharmaceutical composition, comprising an antigen-binding peptide as defined herein, a nucleic acid as defined herein, or a recombinant cell as defined herein, and a pharmaceutically acceptable excipient.

In a further aspect, the present invention relates to an antigen-binding peptide as defined herein, a nucleic acid as defined herein, a recombinant cell as defined herein, or a composition as defined herein, for use in a method of preventing or treating an inflammatory disease, disorder, or condition; wherein, optionally, said inflammatory disease, disorder, or condition is selected from an infection, such as a sepsis; an autoimmune disease, such as rheumatoid arthritis, systemic lupus erythematosus, multiple sclerosis, psoriasis, and inflammatory bowel disease, e.g. Crohn's disease and ulcerative colitis; a neurodegenerative disease; an allergy; an inflammatory medical condition after a transplantation, such as a graft rejection and a graft-versus-host disease (GvHD); a respiratory disease, such as asthma and chronic obstructive pulmonary disease; inflammaging; arthritis, such as activated arthrosis; arteriosclerosis; tendinopathy; an ischemia-perfusion-injury, such as myocardial infarction, ischemic stroke, and hemorrhagic stroke; radiation induced tissue damage; and cancer, such as acute or chronic myeloid leukemia;
wherein, optionally, an anti-inflammatory agent other than the antigen-binding peptide, nucleic acid, recombinant cell, or composition is coadministered with said antigen-binding peptide, nucleic acid, recombinant cell, or composition, preferably selected from nonsteroidal anti-inflammatory drugs, corticosteroids such as cortisone, disease-modifying anti-rheumatic drugs, and antileukotriens, more preferably is coadministered with cortisone.

In a further aspect, the present invention relates to a use of an antigen-binding peptide as defined herein, a nucleic acid as defined herein, a recombinant cell as defined herein, or a composition as defined herein, in a method of detecting BPI in a sample,
wherein, optionally, said method comprises detecting BPI in said sample using an enzyme-linked immunosorbent assay, an immunochromatography, a western blot, and/or an immunohistochemistry;
wherein, preferably, said use is an in vitro use and/or ex vivo use.

In a further aspect, the present invention relates to a method of identifying an agent binding to BPI, preferably an antigen-binding peptide binding to BPI or a small molecule binding to BPI, particularly specifically binding to BPI, comprising
a) providing at least one agent to be tested for a capability of binding, particularly specifically binding, to BPI;
b) testing whether said at least one agent binds to, particularly specifically binds to, an epitope of BPI comprising or consisting of an amino acid sequence selected from

| | |
|---|---|
| FPAAHDRMVYLGLSD | SEQ ID NO: 1; |
| VYLGLSDYFFNTAGLVYQE | SEQ ID NO: 2; |
| PVMEFPAAHDRMVYLGLSD | SEQ ID NO: 3; |
| TFLPEVAKKFPNM | SEQ ID NO: 4; |
| FQTLPVMTKIDSVAGINYLVAP | SEQ ID NO: 5; |
| HYSFYSMDIREFQLP | SEQ ID NO: 102; |
| MVPNVGLKFSISNANIKIS | SEQ ID NO: 103; and |
| QKRFLKMSGNFDLSI | SEQ ID NO: 104. |

In a further aspect, the present invention relates to a method of preventing or treating an inflammatory disease, disorder, or condition, comprising administering an effective amount of an antigen-binding peptide as defined herein, a nucleic acid as defined herein, a recombinant cell as defined herein, or of a composition as defined herein to a patient in need thereof; wherein, optionally, said inflammatory disease, disorder, or condition is selected from an infection, such as a sepsis; an autoimmune disease, such as rheumatoid arthritis, systemic lupus erythematosus, multiple sclerosis, psoriasis, and inflammatory bowel disease, e.g. Crohn's disease and ulcerative colitis; a neurodegenerative disease; an allergy; an inflammatory medical condition after a transplantation, such as a graft rejection and a graft-versus-host disease (GvHD); a respiratory disease, such as asthma and chronic obstructive pulmonary disease; inflammaging; arthritis, such as activated arthrosis; arteriosclerosis; tendinopathy; an ischemia-perfusion-injury, such as myocardial infarction, ischemic stroke, and hemorrhagic stroke; radiation induced tissue damage; and cancer, such as acute or chronic myeloid leukemia;
wherein, optionally, an anti-inflammatory agent other than the antigen-binding peptide, nucleic acid, recombinant cell, or composition is coadministered with said antigen-binding peptide, nucleic acid, recombinant cell, or composition, preferably selected from nonsteroidal anti-inflammatory drugs, corticosteroids such as cortisone, disease-modifying anti-rheumatic drugs, and antileukotriens, more preferably is coadministered with cortisone.

In a further aspect, the present invention relates to the use of an antigen-binding peptide as defined herein, a nucleic acid as defined herein, a recombinant cell as defined herein, or of a composition as defined herein in the manufacture of a medicament for preventing or treating an inflammatory disease, disorder, or condition; wherein, optionally, said inflammatory disease, disorder, or condition is selected from an infection, such as a sepsis; an autoimmune disease, such as rheumatoid arthritis, systemic lupus erythematosus, multiple sclerosis, psoriasis, and inflammatory bowel disease, e.g. Crohn's disease and ulcerative colitis; a neurodegenerative disease; an allergy; an inflammatory medical condition after a transplantation, such as a graft rejection and a graft-versus-host disease (GvHD); a respiratory disease, such as asthma and chronic obstructive pulmonary disease; inflammaging; arthritis such as activated arthrosis; arteriosclerosis; tendinopathy; an ischemia-perfusion-injury, such as myocardial infarction, ischemic stroke, and hemorrhagic stroke; radiation induced tissue damage; and cancer, such as acute or chronic myeloid leukemia.

### DETAILED DESCRIPTION

BPI activates immune cells to produce pro-inflammatory cytokines and chemokines of special importance to the activation of lymphoid cells. Particularly, BPI enhances the immune response in antigen-presenting cells such as dendritic cells to produce cytokines and chemokines that have an immunostimulatory activity. The present invention provides means for inhibiting the pro-inflammatory activity of BPI. The inventors have found that, advantageously, the antigen-binding peptide of the invention inhibits the pro-inflammatory activity of BPI.

In a first aspect, the present invention relates to antigen-binding peptide binding to bactericidal permeability-increasing protein (BPI), wherein said antigen-binding peptide binds to an epitope of BPI comprising or consisting of an amino acid sequence selected from

| | |
|---|---|
| FPAAHDRMVYLGLSD | SEQ ID NO: 1; |
| VYLGLSDYFFNTAGLVYQE | SEQ ID NO: 2; |
| PVMEFPAAHDRMVYLGLSD | SEQ ID NO: 3; |
| TFLPEVAKKFPNM | SEQ ID NO: 4; |
| FQTLPVMTKIDSVAGINYLVAP | SEQ ID NO: 5; |
| HYSFYSMDIREFQLP | SEQ ID NO: 102; |
| MVPNVGLKFSISNANIKIS | SEQ ID NO: 103; and |
| QKRFLKMSGNFDLSI | SEQ ID NO: 104. |

In a preferred embodiment, said antigen-binding peptide binding to BPI is an antigen-binding peptide binding to human BPI, particularly human BPI having a sequence of SEQ ID NO: 105. For example, the epitope may be selected from SEQ ID NO: 1-5. In one embodiment, the antigen-binding peptide is a bispecific antibody or a bispecific antigen-binding fragment thereof, wherein said bispecific antibody or bispecific antigen-binding fragment thereof binds to more than one of SEQ ID NO: 1-5 and 102-104. In one embodiment, the antigen-binding peptide binds to an epitope of BPI comprising or consisting of an amino acid sequence of SEQ ID NO: 1. In one embodiment, the antigen-binding peptide binds to an epitope of BPI comprising or consisting of an amino acid sequence of SEQ ID NO: 2. In one embodiment, the antigen-binding peptide binds to an epitope of BPI comprising or consisting of an amino acid sequence of SEQ ID NO: 3. In one embodiment, the antigen-binding peptide binds to an epitope of BPI comprising or consisting of an amino acid sequence of SEQ ID NO: 4. In one embodiment, the antigen-binding peptide binds to an epitope of BPI comprising or consisting of an amino acid sequence of SEQ ID NO: 5. In one embodiment, the antigen-binding peptide binds to an epitope of BPI comprising or consisting of an amino acid sequence of SEQ ID NO: 102. In one embodiment, the antigen-binding peptide binds to an epitope of BPI comprising or consisting of an amino acid sequence of SEQ ID NO: 103. In one embodiment, the antigen-binding peptide binds to an epitope of BPI comprising or consisting of an amino acid sequence of SEQ ID NO: 104. In one embodiment, the antigen-binding peptide binds to an epitope of BPI comprising or consisting of an amino acid sequence of SEQ ID NO: 102-104. As used herein, the expression "binding/binds to an epitope having SEQ ID NO:" may be used interchangeably with the expression "binding/binds to an epitope having a sequence as defined in SEQ ID NO:".

In one embodiment, said antigen-binding peptide binds to an epitope having SEQ ID NO: 1, e.g. variants 407, 304, 310, 253, and 037. In one embodiment, said antigen-binding peptide binds to an epitope having SEQ ID NO: 2, e.g. variant 41-33. In one embodiment, said antigen-binding peptide binds to an epitope having SEQ ID NO: 3, e.g. variants 064, 234, and 355. In one embodiment, said antigen-binding peptide binds to an epitope having SEQ ID NO: 4, e.g. variant 125. In one embodiment, said antigen-binding peptide binds to an epitope having SEQ ID NO: 5, e.g. variant 230. In one embodiment, said antigen-binding peptide binds to an epitope having a sequence of any one of SEQ ID NO: 102-104, e.g. variant 063.

The abbreviation "BPI", as used herein, relates to bactericidal permeability-increasing protein. BPI is a protein that is part of the innate immune system, coded for in the human by the *BPI* gene. It belongs to the family of lipid-binding serum glycoproteins. The term "antigen-binding peptide", as used herein, relates to a peptide which binds, particularly specifically binds, to an antigen, such as BPI. An antigen-binding molecule may be based on an immunoglobulin, such as an antibody or an antigen-binding fragment thereof, or on a protein scaffold structure having antigen-binding capacity, such as an anticalin protein, an Affilin, an Affimer, an Affitin, an Alphabody, or a DARPin. In a preferred embodiment, the antigen-binding peptide is an antibody or antigen-binding fragment thereof. In a preferred embodiment, the antigen-binding peptide of the present invention is an anti-BPI antibody or an antigen-binding fragment thereof. For example, the antigen-binding peptide may be an antibody or antigen-binding fragment thereof, wherein said antigen-binding fragment may be part of an antibody, e.g. part of a bispecific antibody. The antigen-binding peptide of the invention may bind to, for example, murine or human BPI, preferably to human BPI having the sequence of SEQ ID NO: 105. The term "murine BPI", as mentioned herein, may relate to murine BPI having a sequence of SEQ ID NO: 106. In a preferred embodiment, said antigen-binding peptide, for example said antibody or antigen-binding fragment thereof, specifically binds to BPI. The term "specific" or "specifically binding", as used herein, means that the antigen-binding peptide is capable of binding to an antigen of interest, such as BPI, and does not essentially bind to other antigens; for example, the antigen-binding peptide may be capable of binding to BPI with a KD that is at least ten-fold, preferably at least 100-fold stronger than the KD for binding to other antigens in the human body. Such binding may be exemplified by the specificity of a lock-and-key-principle. In one embodiment, the antigen-binding peptide of the invention is an anti-inflammatory antigen-binding peptide.

There are several classes of antibodies, particularly human antibodies, such as IgG, IgM, IgD and IgE, as well as subclasses, such as IgG1, IgG2, IgG3, and IgG4. Antibodies are typically grouped into classes, also referred to as isotypes, as determined genetically by the constant region. Human constant light chains are classified as kappa (Ck) and lambda (Cl) light chains. Human heavy chains are classified as mu, delta, gamma, alpha, or epsilon, and define the antibody's isotype as IgM, IgD, IgG, IgA, and IgE, respectively. The IgG class is the most commonly used for therapeutic purposes. "IgG", as used herein, relates to a polypeptide belonging to the class of antibodies that are substantially encoded by a recognized immunoglobulin gamma gene. In humans this class comprises subclasses IgG1, IgG2, IgG3, and IgG4. In mice this class comprises subclasses IgG1, IgG2a, IgG2b and IgG3. Thus, "isotype", as used herein, relates to any of the classes or subclasses of immunoglobulins defined by the chemical and antigenic characteristics of their constant regions. The known human immunoglobulin isotypes are IgG1, IgG2, IgG3, IgG4, IgA1, IgA2, IgM, IgD, and IgE. Also useful for the invention may be IgGs that are hybrid compositions of the natural human IgG isotypes. The antigen-binding peptide of the present invention of the present invention may comprise a domain of any of the above mentioned antibody classes, subclasses, and/or chains. The amino acid sequence of the antigen-binding peptide of the present invention, the bispecific molecule of the present invention, and the antigen-binding peptide-drug conjugate of the present invention may be modified by protein engineering, e.g. to comprise constant regions from other immunoglobulin classes mediating improved effector function properties. Such engineered hybrid IgG compositions may provide improved effector function properties, such as improved serum half-life.

An antibody may be, for example, a human antibody, a human-chimeric antibody, a humanized antibody, a chimeric antibody, or a CDR-grafted antibody. In a preferred embodiment, the antibody is an IgG type antibody. The term "IgG type antibody", as used herein, refers to IgG class antibodies, which in humans includes four subclasses (IgG1, IgG2, IgG3, and IgG4). The antibody may thus be an IgG1, IgG2, IgG3, or IgG4 type antibody, preferably is an IgG1 type antibody or a IgG2 type antibody, particularly an IgG1 type antibody, a IgG2a type antibody, or a IgG2b type antibody. The term "peptide", as used herein, may refer to, for example, an oligopeptide, polypeptide, or protein. In one embodiment, the term "antigen-binding peptide" relates to an anti-BPI antibody or an antigen-binding fragment thereof.

The term "antigen-binding fragment", as used herein, may refer to, for example, a Fab, a Fab', a F(ab')2, a scFv, a di-scFv, a V_{H} domain, a single-domain antibody (sdAb), a diabody, a triabody, or a tetrabody. In one embodiment, the antigen-binding fragment of an antibody has the same binding properties as the antibody. In one embodiment, the antigen-binding fragment is selected from a Fab, a Fab', a F(ab')2, a scFv, a di-scFv, a V_{H} domain, a single-domain antibody (sdAb), a diabody, a triabody, and a tetrabody.

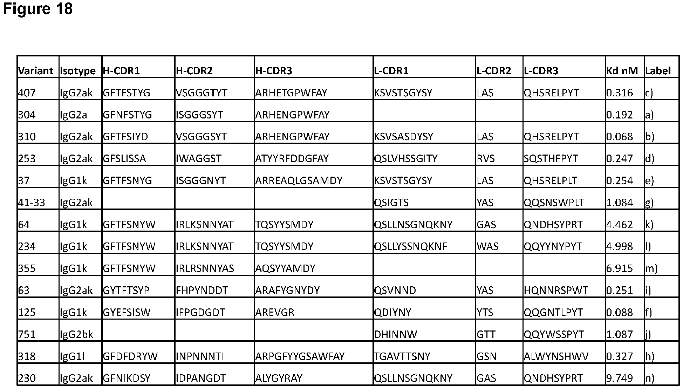

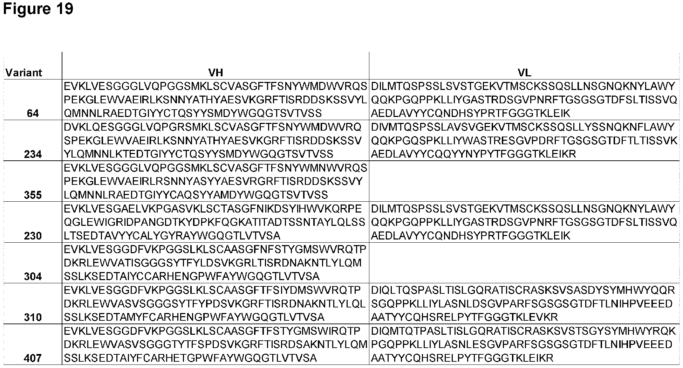

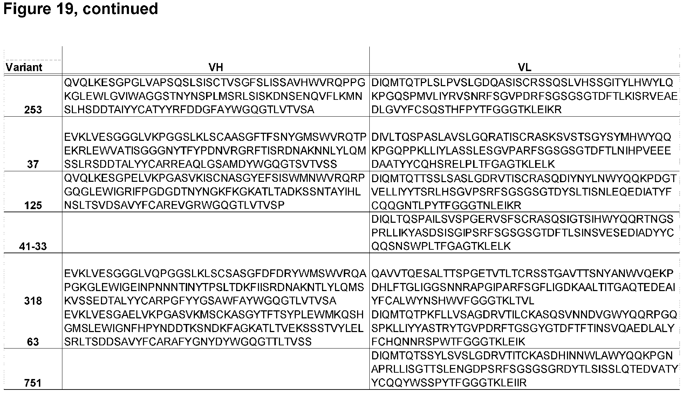

The inventors have surprisingly found that, advantageously, the antigen-binding peptide of the invention, for example variants 407, 304, 310, 253, 037, 41-33, 063, 125, 751, and 318 described herein, neutralize the immunostimulatory activity of BPI. Particularly, the inventors have surprisingly found that, advantageously, the antigen-binding peptide of the invention, for example variants 407, 304, 310, 253, 037, 41-33, 063, 125, 751, and 318 described herein, inhibit a pro-inflammatory activity of BPI, e.g. by inhibiting a production of IL-2. In one embodiment, the antigen-binding peptide of the invention comprises the CDR sequences of any of variants 407, 304, 310, 253, 037, 41-33, 063, 125, 751, and 318, as described herein e.g. in Figure 18; wherein, optionally, the antigen-binding peptide of the invention comprises the CDR sequences of any of variants 407, 310, 253, 037, 063, 125, and 318, as described herein. In one embodiment, the antigen-binding peptide of the invention comprises the VH and/or VL sequences, preferably VH and VL sequences, of any of variants 407, 304, 310, 253, 037, 41-33, 063, 125, 751, and 318, as described herein e.g. in Figure 19; wherein, optionally, the antigen-binding peptide of the invention comprises the VH and/or VL sequences, preferably VH and VL sequences, of any of variants 407, 310, 253, 037, 063, 125, and 318, as described herein.

The inventors have surprisingly found that, advantageously, the antigen-binding peptide of the invention, for example variants 407, 310, 253, 037, 064, 234, 355, 063, 125, 751, and 230 described herein, do not inhibit the antibacterial activity of BPI. In one embodiment, the antigen-binding peptide of the invention comprises the CDR sequences of any of variants 407, 310, 253, 037, 064, 234, 355, 063, 125, 751, and 230, as described herein e.g. in Figure 18; wherein, optionally, the antigen-binding peptide of the invention comprises the CDR sequences of any of variants 407, 310, 253, 037, 064, 234, 063, 125, and 230, as described herein. In one embodiment, the antigen-binding peptide of the invention comprises the VH and/or VL sequences, preferably VH and VL sequences, of any of variants 407, 310, 253, 037, 064, 234, 355, 063, 125, 751, and 230, as described herein e.g. in Figure 19; wherein, optionally, the antigen-binding peptide of the invention comprises the VH and/or VL sequences, preferably VH and VL sequences, of any of variants 407, 310, 253, 037, 064, 234, 063, 125, and 230, as described herein.

The inventors have surprisingly found that, advantageously, the antigen-binding peptides of the invention, e.g. variants 407, 310, 253, 037, 063, 125 and 751, are capable of inhibiting the inflammatory potential of BPI while surprisingly not significantly inhibiting the bactericidal activity of BPI.

In one embodiment, the antigen-binding peptide of the invention comprises the CDR sequences of any of variants 407, 310, 253, 037, 063, 125 and 751, as described herein e.g. in Figure 18; wherein, optionally, the antigen-binding peptide of the invention comprises the CDR sequences of any of variants 407, 310, 253, 037, 063, and 125, as described herein. In one embodiment, the antigen-binding peptide of the invention comprises the VH and/or VL sequences, preferably VH and VL sequences, of any of variants 407, 310, 253, 037, 063, 125 and 751, as described herein e.g. in Figure 19; wherein, optionally, the antigen-binding peptide of the invention comprises the VH and/or VL sequences, preferably VH and VL sequences, of any of variants 407, 310, 253, 037, 063, and 125, as described herein.

In one embodiment, said antigen-binding peptide comprises
a HCDR 1 comprising the amino acid sequence GFX₁FSX₂YX₃ [SEQ ID NO: 150];
   wherein X₁ is any amino acid, preferably an amino acid selected from G, S, T, N, and Q, more preferably an amino acid selected from S, T, N, and Q, even more preferably selected from N and T;
   wherein X₂ is any amino acid, preferably selected from I, A, V, L, M, F, Y, W, T, S, N, and Q, more preferably selected from I, A, V, L, T and S, even more preferably selected from I and T;
   wherein X₃ is any amino acid, preferably selected from D, E, G, A, V, I, L, M, F, Y, and W, more preferably selected from D, E, G, A, V, I, and L, even more preferably selected from D and G;
a HCDR2 comprising the amino acid sequence X₄SGGGX₅YT [SEQ ID NO: 151];
   wherein X₄ is any amino acid, preferably selected from I, V, A, L, M, F, Y, W, and G, more preferably selected from I, V, A, L, and G, even more preferably selected from I and V;
   wherein X₃ is any amino acid, preferably selected from S, T, N, and Q, more preferably selected from S and T;
a HCDR3 comprising the amino acid sequence ARHEX₆GPWFAY [SEQ ID NO: 152];
   wherein X₆ is any amino acid, preferably selected from S, T, N, and Q, more preferably selected from N and T;
a LCDR 1 comprising the amino acid sequence KSVSX₇SX₈YSY [SEQ ID NO: 153];
   wherein X₇ is any amino acid, preferably selected from S, T, N, Q, A, V, I, L, M, F, Y, W, and G, more preferably selected from S, T, N, Q, A, G, V, I, and L, even more preferably selected from A and T;
   wherein X₈ is any amino acid, preferably selected from D, E, A, V, I, L, N, Q, and G, more preferably selected from D, E, G, A, V, I, L, and G, even more preferably selected from D and G;
a LCDR2 comprising the amino acid sequence LAS [SEQ ID NO: 13]; and
a LCDR3 comprising the amino acid sequence QHSRELPYT [SEQ ID NO: 14].

The inventors have surprisingly found that antigen-binding peptides comprising, for example, any of the following combinations of complementarity-determining regions bind to BPI with high affinity:
In one embodiment, said antigen-binding peptide comprises any one of a)-n):
a)
   a heavy chain complementarity-determining region (HCDR) 1 comprising the amino acid sequence GFNFSTYG [SEQ ID NO: 6],
   a HCDR2 comprising the amino acid sequence ISGGGSYT [SEQ ID NO: 7], and
   a HCDR3 comprising the amino acid sequence ARHENGPWFAY [SEQ ID NO: 8]; or
b)
   a HCDR 1 comprising the amino acid sequence GFTFSIYD [SEQ ID NO: 9],
   a HCDR2 comprising the amino acid sequence VSGGGSYT [SEQ ID NO: 10],
   a HCDR3 comprising the amino acid sequence ARHENGPWFAY [SEQ ID NO: 11],
   a LCDR 1 comprising the amino acid sequence KSVSASDYSY [SEQ ID NO: 12],
   a LCDR2 comprising the amino acid sequence LAS [SEQ ID NO: 13], and
   a LCDR3 comprising the amino acid sequence QHSRELPYT [SEQ ID NO: 14]; or
c)
   a HCDR 1 comprising the amino acid sequence GFTFSTYG [SEQ ID NO: 15],
   a HCDR2 comprising the amino acid sequence VSGGGTYT [SEQ ID NO: 16],
   a HCDR3 comprising the amino acid sequence ARHETGPWFAY [SEQ ID NO: 17],
   a LCDR 1 comprising the amino acid sequence KSVSTSGYSY [SEQ ID NO: 18],
   a LCDR2 comprising the amino acid sequence LAS [SEQ ID NO: 19], and
   a LCDR3 comprising the amino acid sequence QHSRELPYT [SEQ ID NO: 20]; or
d)
   a HCDR 1 comprising the amino acid sequence GFSLISSA [SEQ ID NO: 21],
   a HCDR2 comprising the amino acid sequence IWAGGST [SEQ ID NO: 22],
   a HCDR3 comprising the amino acid sequence ATYYRFDDGFAY [SEQ ID NO: 23],
   a LCDR 1 comprising the amino acid sequence QSLVHSSGITY [SEQ ID NO: 24],
   a LCDR2 comprising the amino acid sequence RVS [SEQ ID NO: 25], and
   a LCDR3 comprising the amino acid sequence SQSTHFPYT [SEQ ID NO: 26]; or
e)
   a HCDR 1 comprising the amino acid sequence GFTFSNYG [SEQ ID NO: 27],
   a HCDR2 comprising the amino acid sequence ISGGGNYT [SEQ ID NO: 28],
   a HCDR3 comprising the amino acid sequence ARREAQLGSAMDY [SEQ ID NO: 29],
   a LCDR 1 comprising the amino acid sequence KSVSTSGYSY [SEQ ID NO: 30],
   a LCDR2 comprising the amino acid sequence LAS [SEQ ID NO: 31], and
   a LCDR3 comprising the amino acid sequence QHSRELPLT [SEQ ID NO: 32]; or
f)
   a HCDR 1 comprising the amino acid sequence GYEFSISW [SEQ ID NO: 33],
   a HCDR2 comprising the amino acid sequence IFPGDGDT [SEQ ID NO: 341,
   a HCDR3 comprising the amino acid sequence AREVGR [SEQ ID NO: 35],
   a LCDR 1 comprising the amino acid sequence QDIYNY [SEQ ID NO: 36],
   a LCDR2 comprising the amino acid sequence YTS [SEQ ID NO: 37], and
   a LCDR3 comprising the amino acid sequence QQGNTLPYT [SEQ ID NO: 38]; or
g)
   a LCDR 1 comprising the amino acid sequence QSIGTS [SEQ ID NO: 39],
   a LCDR2 comprising the amino acid sequence YAS [SEQ ID NO: 40], and
   a LCDR3 comprising the amino acid sequence QQSNSWPLT [SEQ ID NO: 41]; or
h)
   a HCDR 1 comprising the amino acid sequence GFDFDRYW [SEQ ID NO: 42],
   a HCDR2 comprising the amino acid sequence INPNNNTI [SEQ ID NO: 43],
   a HCDR3 comprising the amino acid sequence ARPGFYYGSAWFAY [SEQ ID NO: 44],
   a LCDR 1 comprising the amino acid sequence TGAVTTSNY [SEQ ID NO: 45],
   a LCDR2 comprising the amino acid sequence GSN [SEQ ID NO: 46], and
   a LCDR3 comprising the amino acid sequence ALWYNSHWV [SEQ ID NO: 47]; or
i)
   a HCDR 1 comprising the amino acid sequence GYTFTSYP [SEQ ID NO: 48],
   a HCDR2 comprising the amino acid sequence FHPYNDDT [SEQ ID NO: 49],
   a HCDR3 comprising the amino acid sequence ARAFYGNYDY [SEQ ID NO: 50],
   a LCDR 1 comprising the amino acid sequence QSVNND [SEQ ID NO: 51],
   a LCDR2 comprising the amino acid sequence YAS [SEQ ID NO: 52], and
   a LCDR3 comprising the amino acid sequence HQNNRSPWT [SEQ ID NO: 53]; or
j)
   a LCDR 1 comprising the amino acid sequence DHINNW [SEQ ID NO: 54],
   a LCDR2 comprising the amino acid sequence GTT [SEQ ID NO: 55], and
   a LCDR3 comprising the amino acid sequence QQYWSSPYT [SEQ ID NO: 56]; or
k)
   a HCDR 1 comprising the amino acid sequence GFTFSNYW [SEQ ID NO: 57],
   a HCDR2 comprising the amino acid sequence IRLKSNNYAT [SEQ ID NO: 58],
   a HCDR3 comprising the amino acid sequence TQSYYSMDY [SEQ ID NO: 59],
   a LCDR 1 comprising the amino acid sequence QSLLNSGNQKNY [SEQ ID NO: 60],
   a LCDR2 comprising the amino acid sequence GAS [SEQ ID NO: 61], and
   a LCDR3 comprising the amino acid sequence QNDHSYPRT [SEQ ID NO: 62]; or
l)
   a HCDR 1 comprising the amino acid sequence GFTFSNYW [SEQ ID NO: 63],
   a HCDR2 comprising the amino acid sequence IRLKSNNYAT [SEQ ID NO: 64],
   a HCDR3 comprising the amino acid sequence TQSYYSMDY [SEQ ID NO: 65],
   a LCDR 1 comprising the amino acid sequence QSLLYSSNQKNF [SEQ ID NO: 66],
   a LCDR2 comprising the amino acid sequence WAS [SEQ ID NO: 67], and
   a LCDR3 comprising the amino acid sequence QQYYNYPYT [SEQ ID NO: 68]; or
m)
   a HCDR 1 comprising the amino acid sequence GFTFSNYW [SEQ ID NO: 69],
   a HCDR2 comprising the amino acid sequence IRLRSNNYAS [SEQ ID NO: 70], and
   a HCDR3 comprising the amino acid sequence AQSYYAMDY [SEQ ID NO: 71]; or
n)
   a HCDR 1 comprising the amino acid sequence GFNIKDSY [SEQ ID NO: 72],
   a HCDR2 comprising the amino acid sequence IDPANGDT [SEQ ID NO: 73],
   a HCDR3 comprising the amino acid sequence ALYGYRAY [SEQ ID NO: 74],
   a LCDR 1 comprising the amino acid sequence QSLLNSGNQKNY [SEQ ID NO: 75],
   a LCDR2 comprising the amino acid sequence GAS [SEQ ID NO: 76], and
   a LCDR3 comprising the amino acid sequence QNDHSYPRT [SEQ ID NO: 77];

wherein, preferably, the antigen-binding peptide comprises any one of a)-j);
wherein, more preferably, the antigen-binding peptide comprises any one of b)-f), i) and h);
wherein, even more preferably, the antigen-binding peptide comprises any one of b)-f) and i).

In one embodiment, said antigen-binding peptide comprises a CDR, preferably a plurality of CDRs, more preferably 6 CDRs, selected from SEQ ID NO: 6-77.

In one embodiment, said antigen-binding peptide comprises a HCDR 1 having a sequence selected from SEQ ID NO: 6, 9, 15, 21, 27, 33, 42, 48, 57, 63, 69, and 72.

In one embodiment, said antigen-binding peptide comprises a HCDR 2 having a sequence selected from SEQ ID NO: 7, 10, 16, 22, 28, 34, 43, 49, 58, 64, 70, and 73.

In one embodiment, said antigen-binding peptide comprises a HCDR 3 having a sequence selected from SEQ ID NO: 8, 11, 17, 23, 29, 35, 44, 50, 59, 65, 71, and 74.

In one embodiment, said antigen-binding peptide comprises a LCDR 1 having a sequence selected from SEQ ID NO: 12, 18, 24, 30, 36, 39, 45, 51, 54, 60, 66, and 75.

In one embodiment, said antigen-binding peptide comprises a LCDR 2 having a sequence selected from SEQ ID NO: 13, 19, 25, 31, 37, 40, 46, 52, 55, 61, 67, and 76.

In one embodiment, said antigen-binding peptide comprises a LCDR 3 having a sequence selected from SEQ ID NO: 14, 20, 26, 32, 38, 41, 47, 53, 56, 62, 68, and 77.

The inventors have found that surprisingly, the antigen-binding peptide of the present invention does not have any cross-reactivity with murine BPI. In one embodiment, the antigen-binding peptide does not have any cross-reactivity with murine BPI.

In one embodiment, said antigen-binding peptide comprises a HCDR 1 having a sequence of SEQ ID NO: 6, a HCDR2 having a sequence of SEQ ID NO: 7, a HCDR3 having a sequence of SEQ ID NO: 8. In one embodiment, said antigen-binding peptide comprises a HCDR 1 having a sequence of SEQ ID NO: 9, a HCDR2 having a sequence of SEQ ID NO: 10, a HCDR3 having a sequence of SEQ ID NO: 11, a LCDR 1 having a sequence of SEQ ID NO: 12, a LCDR2 having a sequence of SEQ ID NO: 13, and a LCDR3 having a sequence of SEQ ID NO: 14. In one embodiment, said antigen-binding peptide comprises a HCDR 1 having a sequence of SEQ ID NO: 15, a HCDR2 having a sequence of SEQ ID NO: 16, a HCDR3 having a sequence of SEQ ID NO: 17, a LCDR 1 having a sequence of SEQ ID NO: 18, a LCDR2 having a sequence of SEQ ID NO: 19, and a LCDR3 having a sequence of SEQ ID NO: 20. In one embodiment, said antigen-binding peptide comprises a HCDR 1 having a sequence of SEQ ID NO: 21, a HCDR2 having a sequence of SEQ ID NO: 22, a HCDR3 having a sequence of SEQ ID NO: 23, a LCDR 1 having a sequence of SEQ ID NO: 24, a LCDR2 having a sequence of SEQ ID NO: 25, and a LCDR3 having a sequence of SEQ ID NO: 26. In one embodiment, said antigen-binding peptide comprises a HCDR 1 having a sequence of SEQ ID NO: 27, a HCDR2 having a sequence of SEQ ID NO: 28, a HCDR3 having a sequence of SEQ ID NO: 29, a LCDR 1 having a sequence of SEQ ID NO: 30, a LCDR2 having a sequence of SEQ ID NO: 31, and a LCDR3 having a sequence of SEQ ID NO: 32. In one embodiment, said antigen-binding peptide comprises a HCDR 1 having a sequence of SEQ ID NO: 33, a HCDR2 having a sequence of SEQ ID NO: 34, a HCDR3 having a sequence of SEQ ID NO: 35, a LCDR 1 having a sequence of SEQ ID NO: 36, a LCDR2 having a sequence of SEQ ID NO: 37, and a LCDR3 having a sequence of SEQ ID NO: 38. In one embodiment, said antigen-binding peptide comprises a LCDR 1 having a sequence of SEQ ID NO: 39, a LCDR2 having a sequence of SEQ ID NO: 40, and a LCDR3 having a sequence of SEQ ID NO: 41. In one embodiment, said antigen-binding peptide comprises a HCDR 1 having a sequence of SEQ ID NO: 42, a HCDR2 having a sequence of SEQ ID NO: 43, a HCDR3 having a sequence of SEQ ID NO: 44, a LCDR 1 having a sequence of SEQ ID NO: 45, a LCDR2 having a sequence of SEQ ID NO: 46, and a LCDR3 having a sequence of SEQ ID NO: 47. In one embodiment, said antigen-binding peptide comprises a HCDR 1 having a sequence of SEQ ID NO: 48, a HCDR2 having a sequence of SEQ ID NO: 49, a HCDR3 having a sequence of SEQ ID NO: 50, a LCDR 1 having a sequence of SEQ ID NO: 51, a LCDR2 having a sequence of SEQ ID NO: 52, and a LCDR3 having a sequence of SEQ ID NO: 53. In one embodiment, said antigen-binding peptide comprises a LCDR 1 having a sequence of SEQ ID NO: 54, a LCDR2 having a sequence of SEQ ID NO: 55, and a LCDR3 having a sequence of SEQ ID NO: 56. In one embodiment, said antigen-binding peptide comprises a HCDR 1 having a sequence of SEQ ID NO: 57, a HCDR2 having a sequence of SEQ ID NO: 58, a HCDR3 having a sequence of SEQ ID NO: 59, a LCDR 1 having a sequence of SEQ ID NO: 60, a LCDR2 having a sequence of SEQ ID NO: 61, and a LCDR3 having a sequence of SEQ ID NO: 62. In one embodiment, said antigen-binding peptide comprises a HCDR 1 having a sequence of SEQ ID NO: 63, a HCDR2 having a sequence of SEQ ID NO: 64, a HCDR3 having a sequence of SEQ ID NO: 65, a LCDR 1 having a sequence of SEQ ID NO: 66, a LCDR2 having a sequence of SEQ ID NO: 67, and a LCDR3 having a sequence of SEQ ID NO: 68. In one embodiment, said antigen-binding peptide comprises a HCDR 1 having a sequence of SEQ ID NO: 69, a HCDR2 having a sequence of SEQ ID NO: 70, a HCDR3 having a sequence of SEQ ID NO: 71. In one embodiment, said antigen-binding peptide comprises a HCDR 1 having a sequence of SEQ ID NO: 72, a HCDR2 having a sequence of SEQ ID NO: 73, a HCDR3 having a sequence of SEQ ID NO: 74, a LCDR 1 having a sequence of SEQ ID NO: 75, a LCDR2 having a sequence of SEQ ID NO: 76, and a LCDR3 having a sequence of SEQ ID NO: 77. In one embodiment, the antigen-binding peptide of the invention comprises CDR sequences of a variant selected from variants 407, 304, 310, 253, 037, 41-33, 064, 234, 355, 063, 125, 751, 318, and 230, said CDR sequences of said variants being as described in Figure 18.

The terms "complementarity determining region" and "CDR", as used herein, relate to hypervariable or complementarity determining regions (CDRs) of antigen-binding peptides, such as hypervariable or complementarity determining regions (CDRs) found in the variable regions of light or heavy chains of antibodies and antigen-binding fragments thereof. In one embodiment, the terms "hypervariable region" and "complementarity determining region" are used interchangeably. The CDRs may be annotated using IMGT.

In one embodiment, the antigen-binding peptide comprises a heavy chain variable sequence (VH) and a light chain variable sequence (VL); wherein said VH has at least 80%, preferably at least 90%, more preferably at least 95%, even more preferably at least 99%, optionally 100%, sequence identity to an amino acid sequence selected from SEQ ID NO: 78, SEQ ID NO: 79, SEQ ID NO: 81, SEQ ID NO: 83, SEQ ID NO: 85, SEQ ID NO: 87, SEQ ID NO: 90, SEQ ID NO: 92, SEQ ID NO: 95, SEQ ID NO: 97, SEQ ID NO: 99, and SEQ ID NO: 100; and/or wherein said VL has at least 80%, preferably at least 90%, more preferably at least 95%, even more preferably at least 99%, optionally 100%, sequence identity to an amino acid sequence selected from SEQ ID NO: 80, SEQ ID NO: 82, SEQ ID NO: 84, SEQ ID NO: 86, SEQ ID NO: 88, SEQ ID NO: 89, SEQ ID NO: 91, SEQ ID NO: 93, SEQ ID NO: 94, SEQ ID NO: 96, SEQ ID NO: 98, and SEQ ID NO: 101.

In one embodiment, the antigen-binding peptide comprises a heavy chain variable sequence (VH) and a light chain variable sequence (VL). In one embodiment, said VH has at least 80% sequence identity to an amino acid sequence of SEQ ID NO: 78. In one embodiment, said VH has at least 80% sequence identity to an amino acid sequence of SEQ ID NO: 79, and said VL has at least 80% sequence identity to an amino acid sequence of SEQ ID NO: 80. In one embodiment, said VH has at least 80% sequence identity to an amino acid sequence of SEQ ID NO: 81, and said VL has at least 80% sequence identity to an amino acid sequence of SEQ ID NO: 82. In one embodiment, said VH has at least 80% sequence identity to an amino acid sequence of SEQ ID NO: 83, and said VL has at least 80% sequence identity to an amino acid sequence of SEQ ID NO: 84. In one embodiment, said VH has at least 80% sequence identity to an amino acid sequence of SEQ ID NO: 85, and said VL has at least 80% sequence identity to an amino acid sequence of SEQ ID NO: 86. In one embodiment, said VH has at least 80% sequence identity to an amino acid sequence of SEQ ID NO: 87, and said VL has at least 80% sequence identity to an amino acid sequence of SEQ ID NO: 88. In one embodiment, said VL has at least 80% sequence identity to an amino acid sequence of SEQ ID NO: 89. In one embodiment, said VH has at least 80% sequence identity to an amino acid sequence of SEQ ID NO: 90, and said VL has at least 80% sequence identity to an amino acid sequence of SEQ ID NO: 91. In one embodiment, said VH has at least 80% sequence identity to an amino acid sequence of SEQ ID NO: 92, and said VL has at least 80% sequence identity to an amino acid sequence of SEQ ID NO: 93. In one embodiment, said VL has at least 80% sequence identity to an amino acid sequence of SEQ ID NO: 94. In one embodiment, said VH has at least 80% sequence identity to an amino acid sequence of SEQ ID NO: 95, and said VL has at least 80% sequence identity to an amino acid sequence of SEQ ID NO: 96. In one embodiment, said VH has at least 80% sequence identity to an amino acid sequence of SEQ ID NO: 97, and said VL has at least 80% sequence identity to an amino acid sequence of SEQ ID NO: 98. In one embodiment, said VH has at least 80% sequence identity to an amino acid sequence of SEQ ID NO: 99. In one embodiment, said VH has at least 80% sequence identity to an amino acid sequence of SEQ ID NO: 100, and said VL has at least 80% sequence identity to an amino acid sequence of SEQ ID NO: 101. In one embodiment, the antigen-binding peptide of the invention comprises a VH sequence and/or a VL sequence, preferably a VH sequence and a VL sequence, having at least 80% sequence identity to the respective VH and VL sequences of any of the variants described herein, particularly of any of variants 407, 304, 310, 253, 037, 41-33, 064, 234, 355, 063, 125, 751, 318, and 230 as described herein, e.g. in Figure 19. In one embodiment, the antigen-binding peptide of the invention comprises a VH sequence and/or a VL sequence, preferably a VH sequence and a VL sequence, having at least 80% sequence identity to the VH and VL sequence, respectively, of a variant selected from variants 407, 304, 310, 253, 037, 41-33, 064, 234, 355, 063, 125, 751, 318, and 230, said sequence(s) of said variants being as described in Figure 19.

The terms "(X) % sequence identity" and "at least (X) % identical to", as used herein, preferably relate to the percentage of pair-wise identical residues with regard to a homology alignment of a sequence of a polypeptide/nucleic acid of the present invention with a sequence in question. The terms "identity" and "identical", as used herein, refer to a relationship between the sequences of two or more polypeptide molecules or two or more nucleic acid molecules, as determined by aligning and comparing the sequences. Particularly, the expressions "% identity" or "% identical" may refer to the percent of identical residues between the amino acids or nucleotides in the compared molecules and is calculated based on the size of the smallest of the molecules being compared. For these calculations, gaps in alignments (if any) are preferably addressed by a particular mathematical model or computer program (i.e. an algorithm). In calculating percent identity, the sequences being compared are typically aligned in a way that gives the largest match between the sequences. The sequences are typically aligned for optimal matching of their respective amino acid or nucleotide. In one embodiment, an at least 80% sequence identity is an at least 83% sequence identity, preferably an at least 90% sequence identity, more preferably an at least 95% sequence identity, even more preferably an at least 99% sequence identity, optionally a 100% sequence identity.

For example, the antigen-binding peptide of the invention may comprise the CDRs sequences and/or may comprise a VH and VL sequence having at least 80% sequence identity, such as at least 83% sequence identity, to the VH and VL sequence, respectively, of variant(s) as described herein, such as of variant(s) 407, 304, 310, 253, 037, 41-33, 064, 234, 355, 063, 125, 751, 318, and/or 230. In one embodiment, the antigen-binding peptide of the invention comprises the CDRs sequences of a variant selected from 407, 304, 310, 253, 037, 41-33, 063, 125, 751, and 318, optionally selected from 407, 310, 253, 037, 063, 125, and 318. In one embodiment, the antigen-binding peptide of the invention comprises the VH sequence of a variant selected from 407, 304, 310, 253, 037, 064, 234, 355, 063, 125, 318, and 230, e.g. selected from 407, 304, 310, 253, 037, 063, 125, and 318. In one embodiment, the antigen-binding peptide of the invention comprises the VL sequence of a variant selected from 407, 310, 253, 037, 41-33, 064, 234, 063, 125, 751, 318, and 230, e.g. selected from 407, 310, 253, 037, 41-33, 063, 125, 751, and 318. In one embodiment, the antigen-binding peptide of the invention comprises the VH and VL sequences of a variant selected from 407, 310, 253, 037, 064, 234, 063, 125, 318, and 230, e.g. selected from 407, 310, 253, 037, 063, 125, and 318. In one embodiment, the antigen-binding peptide of the invention comprises CDR sequences having at least 80% sequence identity, preferably at least 83% sequence identity, to the CDR sequences of a variant selected from 407, 304, 310, 253, 037, 41-33, 063, 125, 751, and 318, optionally selected from 407, 310, 253, 037, 063, 125, and 318.

Variants 407, 304, 310, 253, 037, 41-33, 064, 234, 355, 063, 125, 751, 318, and 230 are as described in Figures 18 and 19. Particularly, variants 407, 304, 310, 253, 037, 41-33, 064, 234, 355, 063, 125, 751, 318, and 230 have CDR sequences as described in Figure 18. Particularly, variants 407, 304, 310, 253, 037, 41-33, 064, 234, 355, 063, 125, 751, 318, and 230 have VH and/or VL sequences as described in Figure 19. It is meant to be understood that, when referring to an antigen-binding peptide of the invention having CDR sequences of any of the variants described in Figure 18, the respective antigen-binding peptide does not need to have the same isotype and/or KD as the variants shown in Figure 18; particularly, the CDR sequences shown in Figure 18 are meant to be understood as being directly and unambiguously disclosed without being linked to any other feature than the respective CDR sequences; for example, Figure 18 directly and unambiguously discloses an embodiment of the antigen-binding peptide of the invention in which the antigen-binding peptide of the invention has the specific combination of CDR sequences of variant 407, 304, 310, 253, 037, 41-33, 064, 234, 355, 063, 125, 751, 318, or 230.

In one embodiment, said antigen-binding peptide is an antibody or an antigen-binding fragment thereof. In one embodiment, said antigen-binding peptide is selected from an antibody, a Fab, Fab', a F(ab')2, a scFv, di-scFv, a V_{H} domain, a single-domain antibody (sdAb), a diabody, a triabody, and a tetrabody. In one embodiment, the antigen-binding peptide is selected from a humanized antibody, a chimeric antibody, and antigen-binding fragments thereof. An antigen-binding fragment may be, for example, a Fab, Fab', a F(ab')2, a scFv, a di-scFv, a V_{H} domain, a single-domain antibody (sdAb), a diabody, a triabody, or a tetrabody. In one embodiment, said antigen-binding peptide is an IgG2a antibody, an IgG2b antibody, an IgG1 antibody, or an antigen-binding fragment of an IgG2a antibody, of an IgG2b antibody, or of an IgG1 antibody. In one embodiment, said antigen binding peptide is selected from a humanized IgG2a antibody, a humanized IgG2b antibody, a humanized IgG1 antibody, and antigen-binding fragments thereof.

In one embodiment, said antigen-binding peptide neutralizes said BPI. In one embodiment, said antigen-binding peptide neutralizes a pro-inflammatory activity of said BPI. The term "neutralizing", as used herein, may relate to the antigen-binding peptide specifically binding to said BPI and blocking the pro-inflammatory activity of BPI, particularly blocking the ability of BPI to induce a production of pro-inflammatory cytokines, such as IL-2. In one embodiment, said antigen-binding peptide inhibits a pro-inflammatory activity of BPI in immune cells, particularly dendritic cells, leukocytes, and/or macrophages. In a preferred embodiment, said antigen-binding peptide inhibits an induction of cytokines, particularly IL-2 and/or TNF, by BPI in immune cells, particularly in dendritic cells, leukocytes, and/or macrophages, such as in bone marrow-derived dendritic cells. The term "immune cell", as used herein, relates to any of the cells involved in the immune system of an organism. These include any of the cells of the innate immune system and the adapted immune system, for example, cytotoxic T cells, T helper cells, regulatory T cells, γδ T cells, NKT cells, NK cells, B lymphocytes, innate lymphoid cells, macrophages, monocytes, neutrophils, dendritic cells, mast cells, eosinophils, and basophils. For example, the immune cells may be dendritic cells, leukocytes, and/or macrophages. The inventors have found that, advantageously, the antigen-binding peptide of the invention inhibits a pro-inflammatory activity of BPI in immune cells, particularly dendritic cells, leukocytes, and/or macrophages. The term "pro-inflammatory activity", as used herein, relates to an activity of promoting inflammation, such as by promoting a production of cytokines which are involved in inflammation. The expression "induction of cytokines", as used herein, may relate to promoting the production of the cytokines. In one embodiment, said antigen-binding peptide does not inhibit an antibiotic activity of BPI, particularly a bactericidal activity of BPI. In one embodiment, said antigen-binding peptide inhibits a pro-inflammatory activity of BPI and does not inihbit an antibiotic activity of BPI, particularly a bactericidal activity of BPI.

In one embodiment, said antigen-binding peptide binds to BPI, particularly human BPI, with a KD of 10 nM or less, preferably 7 nM or less, more preferably 1.5 nM or less, even more preferably 1.1 nM or less, still even more preferably 0.5 nM or less, still even more preferably 0.3 nM or less, such as 0.01 nM or less. In one embodiment, said KD is measured by an enzyme-linked immunosorbent assay (ELISA). The term "KD", as used herein, relates to the dissociation constant, which is the inverse of the association constant. In one embodiment, said antigen-binding peptide binds to human BPI with a KD of 10 nM or less, preferably 7 nM or less, as determined using an ELISA. In a preferred embodiment, the KD is determined using an ELISA.

In a further aspect, the present invention relates to a nucleic acid, particularly an isolated nucleic acid, encoding an antigen-binding peptide as defined herein. The term "nucleic acid", as used herein, relates to a nucleotide sequence, such as ribonucleic acid or deoxyribonucleic acid. The term "isolated nucleic acid", as used herein, preferably relates to nucleic acids which are separated from constituents, such as cellular constituents, which the nucleic acids are normally associated with in nature; for example, the isolated nucleic acid is at least 80%, 90%, 95% pure by weight, i.e. devoid of contaminating constituents. For example, the isolated nucleic acid may be a DNA molecule that is separated from sequences with which it is immediately contiguous (in the 5' and 3' directions) in a naturally occurring genome of an organism from which it was derived. For example, the isolated nucleic acid may be a DNA molecule inserted into an expression vector, such as a plasmid or a viral vector, or integrated into the genomic DNA of a prokaryote or eukaryote. Accordingly, the present invention also provides expression vectors which comprise the nucleic acid of the invention. The nucleic acid of the invention may be used in the manufacture of the antigen-binding peptide of the invention, particularly by means of expression in a cell culture.

In a further aspect, the present invention relates to a recombinant cell comprising a nucleic acid as defined herein, for example an isolated nucleic acid provided in an expression vector, and its use in the manufacture of the antigen-binding peptide of the invention. The recombinant cell may be, for example, a yeast cell, an insect cell, or a mammalian cell. For example, the recombinant cell may be a yeast cell selected from *Saccharomyces cerevisiae, Hansenula polymorpha, Schizosaccharomyces pombe, Schwanniomyces occidentalis, Kluyveromyceslactis, Yarrowia lipolytica* and *Pichia pastoris;* an insect cell selected from Sf9, Sf21, S2, Hi5, and BTI-TN-5B1-4 cells; or a mammalian cell selected from HEK293, HEK293T, HEK293E, HEK 293F, NS0, per.C6, MCF-7, HeLa, Cos-1, Cos-7, PC-12, 3T3, Vero, vero-76, PC3, U87, SAOS-2, LNCAP, DU145, A431, A549, B35, H1299, HUVEC, Jurkat, MDA-MB-231, MDA-MB-468, MDA-MB-435, Caco-2, CHO, CHO-K1, CHO-B11, CHO-DG44, BHK, AGE1.HN, Namalwa, WI-38, MRC-5, HepG2, L-929, RAB-9, SIRC, RK13, 11B11, 1D3, 2.4G2, A-10, B-35, C-6, F4/80, IEC-18, L2, MH1C1, NRK, NRK-49F, NRK-52E, RMC, CV-1, BT, MDBK, CPAE, MDCK.1, MDCK.2, and D-17.

In a further aspect, the present invention relates to a composition, preferably a pharmaceutical composition, comprising an antigen-binding peptide as defined herein, a nucleic acid as defined herein, or a recombinant cell as defined herein, and a pharmaceutically acceptable excipient. Pharmaceutically acceptable excipients, for example as comprised by a composition of the invention, may be selected from carriers, diluents, fillers, binders, lubricants, disintegrants, glidants, colorants, pigments, taste masking agents, sweeteners, flavorants, plasticizers, and any acceptable auxiliary substances such as absorption enhancers, penetration enhancers, surfactants, co-surfactants, and specialized oils. Suitable pharmaceutically acceptable excipient(s) may, for example, be selected based on the dosage form, the intended mode of administration, the intended release rate, and manufacturing reliability. Examples of common types of pharmaceutically acceptable excipient(s) include, for example, polymers, waxes, calcium phosphates, and sugars.

In a further aspect, the present invention relates to an antigen-binding peptide as defined herein, a nucleic acid as defined herein, a recombinant cell as defined herein, or a composition as defined herein, for use in a method of preventing or treating an inflammatory disease, disorder, or condition. In one embodiment, said inflammatory disease, disorder, or condition is selected from an infection, such as a sepsis; an autoimmune disease, such as rheumatoid arthritis, systemic lupus erythematosus, multiple sclerosis, psoriasis, and inflammatory bowel disease, e.g. Crohn's disease and ulcerative colitis; a neurodegenerative disease; an allergy; an inflammatory medical condition after a transplantation, such as a graft rejection and a graft-versus-host disease (GvHD); a respiratory disease, such as asthma and chronic obstructive pulmonary disease; inflammaging; arthritis, such as activated arthrosis; arteriosclerosis; tendinopathy; an ischemia-perfusion-injury, such as myocardial infarction, ischemic stroke, and hemorrhagic stroke; radiation induced tissue damage; and cancer, such as acute or chronic myeloid leukemia. In one embodiment, an anti-inflammatory agent other than the antigen-binding peptide, nucleic acid, recombinant cell, or composition is coadministered with said antigen-binding peptide, nucleic acid, recombinant cell, or composition, preferably selected from nonsteroidal anti-inflammatory drugs, corticosteroids such as cortisone, disease-modifying anti-rheumatic drugs, and antileukotriens, more preferably is coadministered with cortisone. For example, the disease-modifying anti-rheumatic drugs, as mentioned herein in the context of any of the aspects of the invention, may be selected from hydroxychloroquine, leflunomide, methotrexate, mycophenolate, sulfasalazine, tofacitinib, and baricitinib. Arthritis may be selected from, for example, rheumatoid arthritis, gout, and ostheoarthritis such as activated arthrosis; optionally selected from arthritis other than rheumatoid arthritis, particularly selected from gout and ostheoarthritis such as activated arthrosis.

In a further aspect, the present invention relates to a use of an antigen-binding peptide as defined herein, a nucleic acid as defined herein, a recombinant cell as defined herein, or a composition as defined herein, in a method of detecting BPI in a sample. In a preferred embodiment, said method of detecting BPI in a sample is a method of detecting human BPI in a sample, preferably human BPI having at least 80%, preferably 90%, more preferably 95%, even more preferably 98% or 99%, sequence identity to a sequence of SEQ ID NO: 105, more preferably human BPI having a sequence of SEQ ID NO: 105. In one embodiment, said method of detecting BPI in a sample comprises detecting BPI in said sample using an enzyme-linked immunosorbent assay, an immunochromatography, a western blot, and/or an immunohistochemistry. For example, said method of detecting BPI in a sample may comprise detecting immune complexes comprising BPI and an anti-BPI-autoantibody in a sample of a patient. In a preferred embodiment, said use is an in vitro use and/or ex vivo use. In one embodiment, said method of detecting BPI in a sample comprises detecting an anti-BPI autoantibody in said sample. The antigen-binding peptide of the invention, the nucleic acid of the invention, the recombinant cell of the invention, or the composition of the invention may, for example, be used for diagnostic purposes to detect BPI in a patient sample. The sample may be, for example, a tissue sample and/or cell sample of a patient. Detecting BPI in a sample may comprise contacting the sample with the antigen-binding peptide of the invention, the nucleic acid of the invention, the recombinant cell of the invention, or the composition of the invention under conditions that allow specific binding to BPI and subsequently detecting the antigen-binding peptide of the invention, the nucleic acid of the invention, the recombinant cell of the invention, or the composition of the invention, such as by means of detecting a fluorophore or a chemiluminescent agent.

In a further aspect, the present invention relates to a method of identifying an agent binding to BPI, preferably an antigen-binding peptide binding to BPI or a small molecule binding to BPI, particularly specifically binding to BPI, comprising
a) providing at least one agent to be tested for a capability of binding, particularly specifically binding, to BPI;
b) testing whether said at least one agent binds to, particularly specifically binds to, an epitope of BPI comprising or consisting of an amino acid sequence selected from

| | |
|---|---|
| FPAAHDRMVYLGLSD | SEQ ID NO: 1; |
| VYLGLSDYFFNTAGLVYQE | SEQ ID NO: 2; |
| PVMEFPAAHDRMVYLGLSD | SEQ ID NO: 3; |
| TFLPEVAKKFPNM | SEQ ID NO: 4; |
| FQTLPVMTKIDSVAGINYLVAP | SEQ ID NO: 5; |
| HYSFYSMDIREFQLP | SEQ ID NO: 102; |
| MVPNVGLKFSISNANIKIS | SEQ ID NO: 103; and |
| QKRFLKMSGNFDLSI | SEQ ID NO: 104. |

In a preferred embodiment, the method of identifying an agent binding to BPI is a method of identifying an agent binding to human BPI, for example an agent binding to human BPI having a sequence of SEQ ID NO: 105. In one embodiment, said method is a screening method. In one embodiment, said at least one agent is a library of agents, particularly a library comprising a plurality of agents, preferably a plurality of antigen-binding peptides, particularly antibodies or antigen-binding fragments thereof, or a plurality of small molecules. In one embodiment, said method of identifying comprises a step of identifying an agent binding to BPI, if said at least one agent binds to, preferably specifically binds to, an epitope of BPI comprising or consisting of an amino acid sequence selected from SEQ ID NO: 1; SEQ ID NO: 2; SEQ ID NO: 3; SEQ ID NO: 4; SEQ ID NO: 5; SEQ ID NO: 102; SEQ ID NO: 103; and SEQ ID NO: 104. In one embodiment, said agent is an antigen-binding peptide as defined herein. In one embodiment, said agent is an antigen-binding peptide, preferably an antibody or an antigen-binding fragment thereof, or a small molecule. The agent identified using the method of identifying an agent may be used in the prevention or treatment of an inflammatory disease, disorder, or condition, as defined herein in the context of any of the aspects of the invention. For example, agent identified using the method of identifying an agent may be for use in a method of preventing or treating an inflammatory disease, disorder, or condition as defined herein.

In a further aspect, the present invention relates to a method of preventing or treating an inflammatory disease, disorder, or condition, comprising administering an effective amount of an antigen-binding peptide of the invention, a nucleic acid of the invention, a recombinant cell of the invention, or of a composition of the invention to a patient in need thereof. In one embodiment, said inflammatory disease, disorder, or condition is selected from an infection, such as a sepsis; an autoimmune disease, such as rheumatoid arthritis, systemic lupus erythematosus, multiple sclerosis, psoriasis, and inflammatory bowel disease, e.g. Crohn's disease and ulcerative colitis; a neurodegenerative disease; an allergy; an inflammatory medical condition after a transplantation, such as a graft rejection and a graft-versus-host disease (GvHD); a respiratory disease, such as asthma and chronic obstructive pulmonary disease; inflammaging; arthritis, such as activated arthrosis; arteriosclerosis; tendinopathy; an ischemia-perfusion-injury, such as myocardial infarction, ischemic stroke, and hemorrhagic stroke; radiation induced tissue damage; and cancer, such as acute or chronic myeloid leukemia. In one embodiment, an anti-inflammatory agent other than the antigen-binding peptide, nucleic acid, recombinant cell, or composition is coadministered with said antigen-binding peptide, nucleic acid, recombinant cell, or composition, preferably selected from nonsteroidal anti-inflammatory drugs, corticosteroids such as cortisone, disease-modifying anti-rheumatic drugs, and antileukotriens, more preferably is coadministered with cortisone. For example, the antigen-binding peptide, nucleic acid, recombinant cell, or composition may be administered to a patient in need thereof intravenously, intravascularly, orally, nasally, mucosally, intrabronchially, intrapulmonarily, intradermally, subcutaneously, intramuscularly, intravascularly, intraperitoneally, intrathecally, intracerebrally, intracranially, intraocularly, intraarticularly, intracapsulary, intratendinously or intranodally. The terms "effective amount" and "therapeutically effective amount", as used herein, relate to an amount of an antigen-binding peptide of the invention, a nucleic acid of the invention, a recombinant cell of the invention, or of a composition of the invention, wherein said amount has an anti-inflammatory effect in a patient. In one embodiment, an effective amount is an amount of an antigen-binding peptide of the invention, a nucleic acid of the invention, a recombinant cell of the invention, or of a composition of the invention, which inhibits a pro-inflammatory activity of BPI in immune cells, e.g. immune cells in vivo. In one embodiment, an effective amount of an antigen-binding peptide of the invention, a nucleic acid of the invention, a recombinant cell of the invention, or of a composition of the invention in vivo is in the range of from 1 pg/kg/dose to 1 g/kg/dose, preferably 100 pg/kg/dose to 300 mg/kg/dose, more preferably 1 ng/kg/dose to 30 mg/kg/dose.

In a further aspect, the present invention relates to the use of an antigen-binding peptide as defined herein, a nucleic acid as defined herein, a recombinant cell as defined herein, or of a composition as defined herein in the manufacture of a medicament for preventing or treating an inflammatory disease, disorder, or condition; wherein, optionally, said inflammatory disease, disorder, or condition is selected from an infection, such as a sepsis; an autoimmune disease, such as rheumatoid arthritis, systemic lupus erythematosus, multiple sclerosis, psoriasis, and inflammatory bowel disease, e.g. Crohn's disease and ulcerative colitis; a neurodegenerative disease; an allergy; an inflammatory medical condition after a transplantation, such as a graft rejection and a graft-versus-host disease (GvHD); a respiratory disease, such as asthma and chronic obstructive pulmonary disease; inflammaging; arthritis, such as activated arthrosis; arteriosclerosis; tendinopathy; an ischemia-perfusion-injury, such as myocardial infarction, ischemic stroke, and hemorrhagic stroke; radiation induced tissue damage; and cancer, such as acute or chronic myeloid leukemia. For example, the antigen-binding peptide of the invention, the nucleic acid of the invention, the recombinant cell of the invention, or the composition of the invention may be formulated into a pharmaceutical composition for administration to a patient in need thereof, such as a patient suffering from or being at risk of acquiring an inflammatory disease, disorder, or condition.

The terms "immunostimulatory" and "pro-inflammatory", as used herein, may be used interchangeably. In one embodiment, the term "patient" relates to a human or an animal, preferably a human. In one embodiment, the patient is a patient suffering from or being at risk of acquiring a bacterial infection such as an infection with a Gram-negative bacterium. The terms "of the [present] invention", "in accordance with the invention", "according to the invention" and the like, as used herein are intended to refer to all aspects and embodiments of the invention described and/or claimed herein. As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated. The term "about", as used herein in the context of numbers or data, intends to include deviations (±) which usually are to be considered in the respective technical field.

### BRIEF DESCRIPTION OF THE FIGURES

The present invention is now further described by reference to the following figures.

All methods mentioned in the figure descriptions below were carried out as described in detail in the examples.

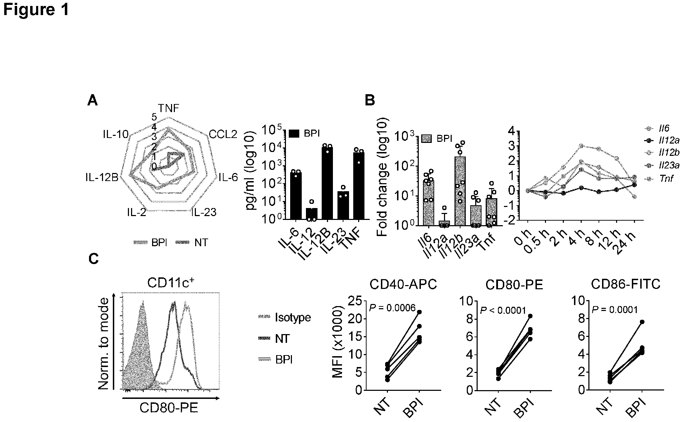

**Figure 1****. BPI activates and maturates murine BMDCs. (A)** Cytokine levels measured in the cell supernatant after BPI-stimulation compared to untreated cells (n = 3). **(B)** Gene expression after BPI-stimulation for 4 h (n = 7) and in one kinetic approach. **(C)** Mean fluorescence intensity (MFI) of CD40, CD80 and CD86 after stimulation with BPI compared to untreated cells (NT; n = 4). Cytokine levels in the spider diagram are presented as pg/ml in a logarithmic scale (A). Results are shown as means ± SEM (A, B). Representative histograms and summarized data of flow cytometric analysis of CD11c+ BMDCs are shown (C). Statistical testing was performed using Student's ratio paired t-test.

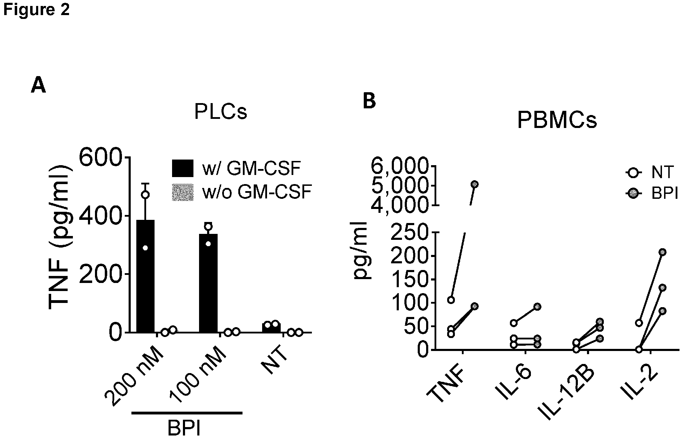

**Figure 2****. BPI is mediator of inflammation in various murine and human immune cells. (A)** Murine peritoneal lavage cells (PLCs) were cultivated in the presence of GM-CSF for 7 days. Secretion of TNF was measured 18 h after stimulation with BPI in concentrations as indicated. Results for PLCs not treated with BPI (NT) are also depicted. Data are shown from two biological replicates. **(B)** TNF, IL-6, IL-12p40 and IL-2 secretion of PBMCs in response to stimulation with BPI (200 nM) for 24 h after priming with recombinant GM-CSF (80 ng/ml) for 24 h. Data are shown for three individual healthy blood donors.

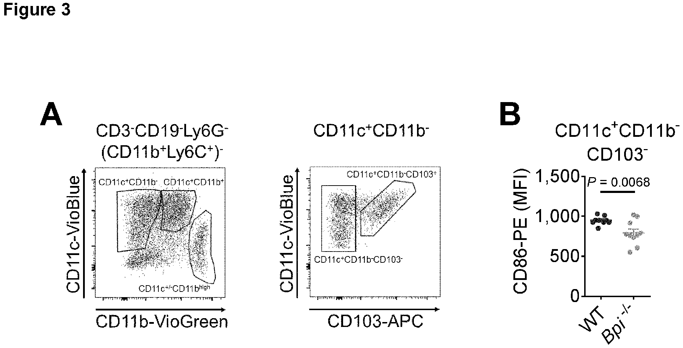

**Figure 3****. BPI induces immune cell activation in vivo.** Dendritic cell (DC) immunophenotyping of C57BL/6J WT and *Bpi*^{*-*/*-*} mice co-housed in a conventional animal facility (n = 10). **(A)** Flow cytometric gating strategy of DCs derived from mesenterial lymph nodes in CD3⁻CD19⁻Ly6G⁻(CD11b⁺Ly6C⁺)⁻cell subsets. **(B)** Expression of CD86 in CD11c⁺CD11b⁻CD103⁻ DCs. Data are depicted as box plots showing median, upper and lower quartiles and whiskers indicating minimal and maximal values. Statistical testing was performed using unpaired Student's t test.

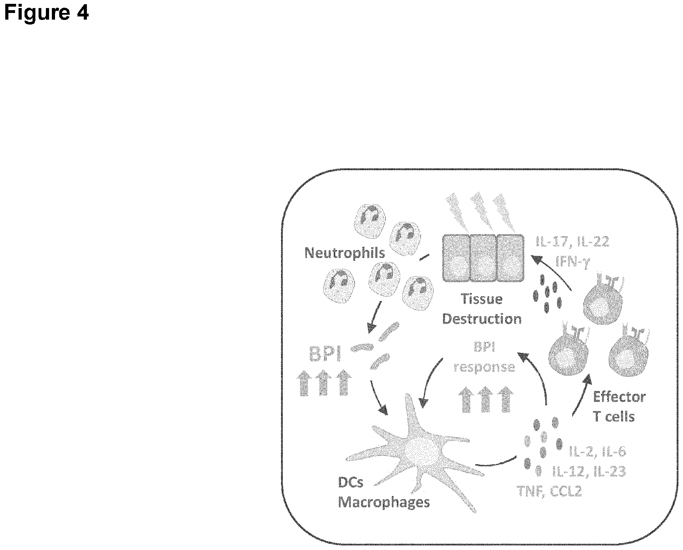

**Figure 4** Chronic inflammation results in several BPI-dependent positive feedback loops and thus an uncontrolled amplification of inflammation:
   1. In the physiological state, DCs, macrophages and other immune cells do not exhibit BPI. This only arises through inflammatory stimuli, e.g. in the context of tissue damage. *In vitro,* this BPI responsiveness can be induced by pro-inflammatory cytokines such as TNF, IL-18 or GM-CSF in celltypes such as DCs (Figure 1), macrophages (Figure 2A) and also human peripheral mononuclear cells (PBMCs; Figure 2B). Thus, the cytokines released during inflammation induce BPI-responsiveness in DCs, macrophages and other immune cells, which then produce more pro-inflammatory cytokines, so that BPI-responsiveness is maintained or even increased.
   2. The stimulation of BPI-responsive cells leads to the release of cytokines, such as IL-2, IL-6, IL-12 and TNF, which can induce the activation of T cells and thus cause direct tissue destruction, but also induce the formation of IL-17 and IL-22. Both factors cause a further influx of neutrophils, resulting in even more BPI entering the tissue.

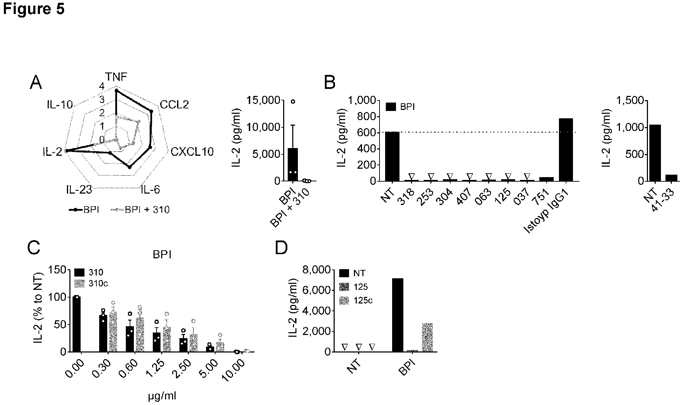

**Figure 5****. Inflammatory potential of BPI can be dampened by BPI-specific antibodies.** Murine bone marrow-derived dendritic cells (BMDCs) pre-incubated with indicated concentrations of a neutralizing αBPI antibody and stimulated with BPI (200 nM). **(A)** TNF, CCL2, CXCL10, IL-6, IL-23, IL-2 levels (pg/ml) as exemplified for the use of antibody 310 (10 µg/ml) are presented as in spider diagrams using a logarithmic scale. BPI (bold black line) or BPI in combination with aBPI antibody 310 (10 µg/ml; bold grey line) for 18 h are indicated (left graph). Results for interleukin-2 (IL-2) secretion are depicted separately (right graph; mean ± SEM). **(B)** Experimental set-up as described in (A) using 9 different aBPI antibodies. Reductions of interleukin-2 (IL-2) secretion by the antibodies (10 µg/ml) are indicated. Arrow heads indicate very low IL-2 levels. **(C)** Comparison of the murine aBPI antibody 310 and the respective chimeric aBPI antibody c310 regarding the relative reduction of BPI-mediated IL-2 secretion (mean ± SEM). **(D)** Comparison of the murine aBPI antibody 125 and the respective chimeric aBPI antibody c125 regarding the reduction of BPI-mediated IL-2 secretion. Data are shown for three (A, C) or one (B, D) biological replicates. Advantageously, the antibodies of the invention inhibit the immunostimulatory effect of BPI.

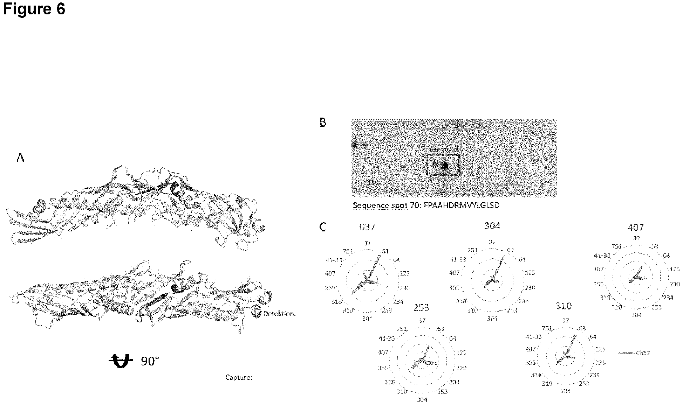

**Figure 6****. Epitope depiction of antibodies 037, 253, 304, 310 and 407. (A)** Cartoon representation of the epitope bound by named antibodies. **(B)** Definition of the bound epitope as exemplified for aBPI antibody 310 on a membrane spotted with overlapping peptides derived from BPI. Sequence 70 was bound by the antibodies. The respective amino acid sequence of the epitope is indicated. Therefore, the sequence bound is FPAAHDRMVYLGLSD. **(C)** Spider diagrams binding of the indicated antibody (037, 253, 304, 310 and 407 as indicated in the heading) to BPI when bound to the respective antibodies as measured by Luminex technology.

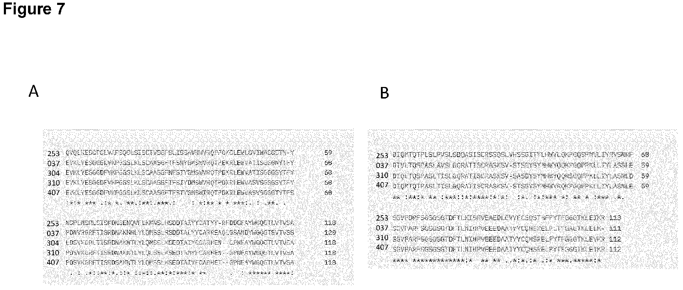

**Figure 7****. Sequence alignment of antibodies 037, 253, 304, 310 and 407. (A)** Sequences of the heavy chains. **(B)** Sequences of the light chains. Stars *, colons and dots indicate similarities within the sequences. Alignment was performed using CLUSTAL 0(1.2.4) multiple sequence alignment.

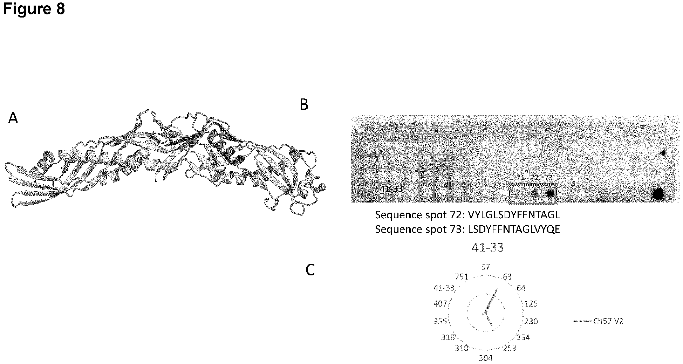

**Figure 8****. Epitope depiction of antibody 41-33. (A)** Cartoon representation of the epitope bound by named antibody. **(B)** Definition of the bound epitope of aBPI antibody 41-33 on a membrane spotted with overlapping peptides derived from BPI. Sequences 72 and 73 were bound by the antibody. The respective amino acid sequences are indicated. Therefore, the sequence bound is VYLGLSDYFFNTAGLVYQE. **(C)** Spider diagram binding of the indicated antibody (41-33 as indicated in the heading) to BPI when bound to the respective antibodies as measured by Luminex technology.

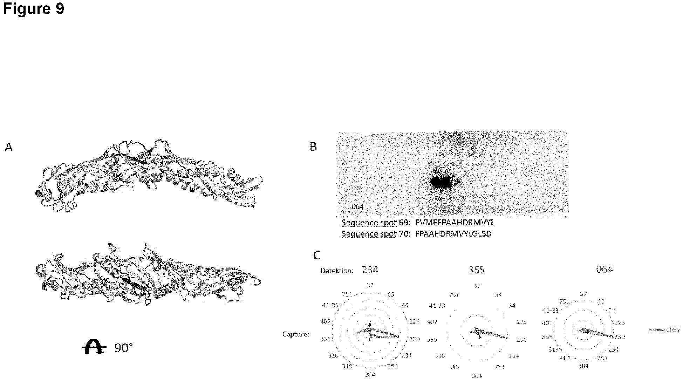

**Figure 9****. Epitope depiction of antibodies 064, 234 and 355. (A)** Cartoon representation of the epitope bound by named antibodies. **(B)** Definition of the bound epitope as exemplified for aBPI antibody 064 on a membrane spotted with overlapping peptides derived from BPI. Sequence 69 and 70 was bound by the antibodies. The respective amino acid sequences of the epitope are indicated. Therefore, the sequence bound is PVMEFPAAHDRMVYLGLSD. **(C)** Spider diagrams binding of the indicated antibody (234, 355 and 064 as indicated in the heading) to BPI when bound to the respective antibodies as measured by Luminex technology.

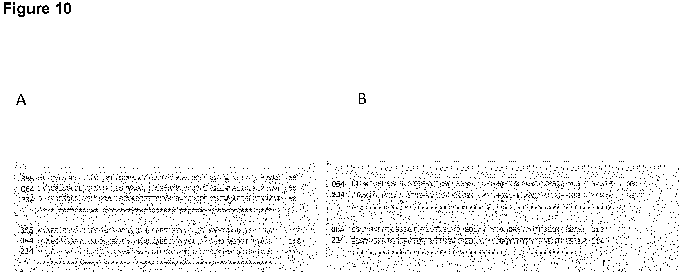

**Figure 10****. Sequence alignment of antibodies 064, 234 and 355. (A)** Sequences of the heavy chains. **(B)** Sequences of the light chains. Stars *, colons and dots indicate similarities within the sequences. Alignment was performed using CLUSTAL 0(1.2.4) multiple sequence alignment.

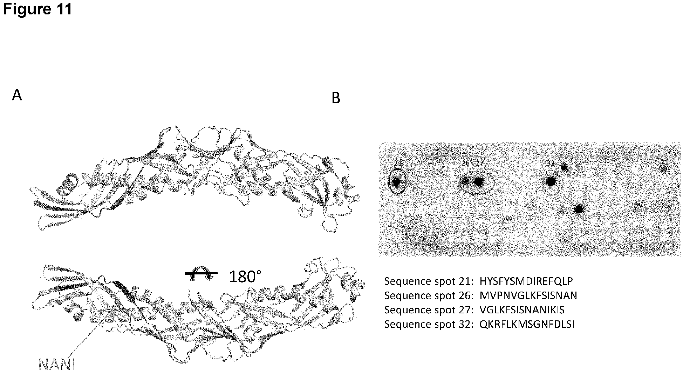

**Figure 11****. Epitope depiction of antibody 063. (A)** Cartoon representation of the epitope bound by named antibody. **(B)** Definition of the bound epitope of aBPI antibody 063 on a membrane spotted with overlapping peptides derived from BPI. Sequences 21, 26, 27 and 32 were bound by the antibody. Sequences are not linear ordered indicating that the binding of the antibody to BPI depends on the conformation of the protein. The respective amino acid sequences are indicated. Therefore, the sequences bound are HYSFYSMDIREFQLP, MVPNVGLKFSISNANIKIS and QKRFLKMSGNFDLSI.

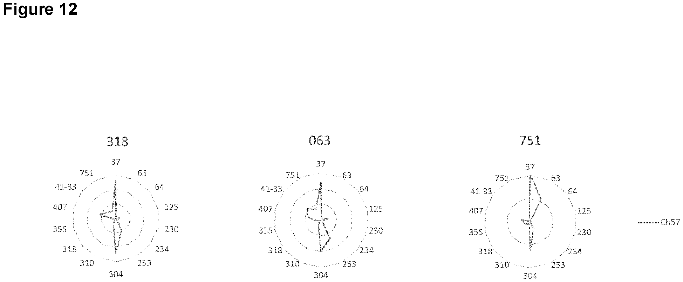

**Figure 12****. Epitope prediction of antibody 318 and 751.** Spider diagrams binding of the indicated antibody (318, 063 and 751 as indicated in the heading) to BPI when bound to the respective antibodies as measured by Luminex technology. Both Antibodies 318 and 751 show a similar binding pattern as antibody 063.

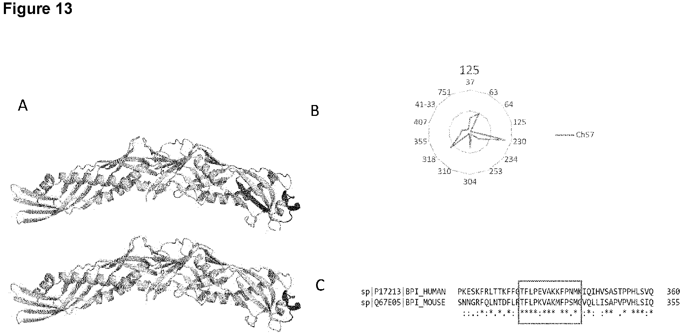

**Figure 13****. Epitope depiction of antibody 125. (A)** Cartoon representation of the epitope bound by named antibody. **(B)** Spider diagram binding of the indicated antibody (125 as indicated in the heading) to BPI when bound to the respective antibodies as measured by Luminex technology. **(C)** Antibody 125 binds to human and murine BPI. Only limited sequences are highly similar in their amino acid sequence. We found by sequence alignment that TFLPEVAKKFPNM of human BPI and TFLPEVAKMFPSM of murine BPI are highly similar. Sequence alignment of human BPI and murine BPI at the epitope site is shown. Therefore, the sequence bound by human BPI is TFLPEVAKKFPNM.

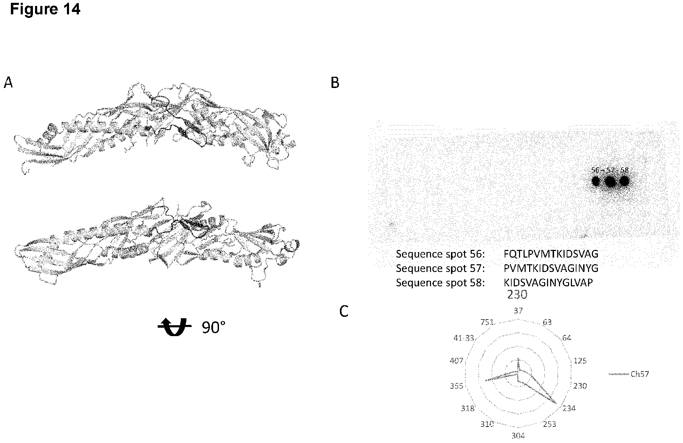

**Figure 14****. Epitope depiction of antibody 230. (A)** Cartoon representation of the epitope bound by named antibody. **(B)** Definition of the bound epitope of aBPI antibody 230 on a membrane spotted with overlapping peptides derived from BPI. Sequences 56, 57 and 58 were bound by the antibody. The respective amino acid sequences are indicated. The sequence bound is FQTLPVMTKIDSVAGINYLVAP. **(C)** Spider diagram binding of the indicated antibody (230 as indicated in the heading) to BPI when bound to the respective antibodies as measured by Luminex technology.

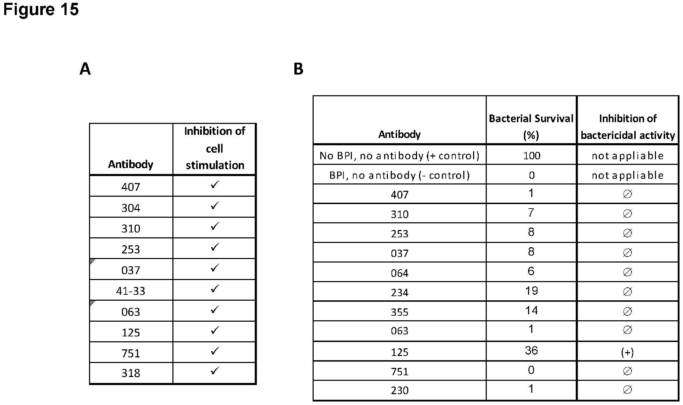

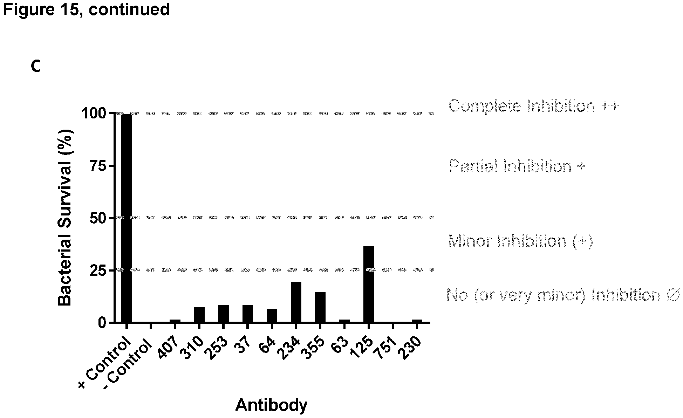

**Figure 15****. Inhibition of the bactericidal activity of BPI by the antibodies. (A)** Table indicating the inhibition of the immunostimulatory potential of BPI as already indicated in Figure 5. **(B)** For determination of the bactericidal activity of BPI dose-response experiments were performed. *E. coli* DH10B was incubated with BPI to cause complete killing. Bacteria surviving are indicated when BPI was pre-incubated with the respective antibody (4 µM). BPI was used at a final concentration of 1 nM with exception of antibodies 063 and 751 where a final concentration of 2 nM BPI was used, i.e. the antibody concentration was exceeding BPI concentration by 4,000 and 2,000 fold. For the positive and negative control no antibody and no or 2 nM BPI was added, respectively. **(C)** Diagram depicting the bacterial survival. Discontinuous lines indicate the cut-off for interpretation of the extent of inhibition. Abbreviations: ✔ inhibition of pro-inflammatory activity, Ø no (or very minor) inhibition of bactericidal activity, ++ complete inhibition of bactericidal activity, + partial inhibition of bactericidal activity, (+) minor inhibition of bactericidal activity. Advantageously, the antigen-binding peptides of the invention inhibit the immunostimulatory activity of BPI and do not inhibit the bactericidal activity of BPI. Antigen-binding peptides showing "(+) minor inhibition" may be considered as showing no inhibition. Antigen-binding peptides showing an inhibition of the immunostimulatory potential of BPI and showing only minor, preferably very minor, more preferably no inhibition, of the bactericidal activity of BPI are preferred for a clinical application in a patient.

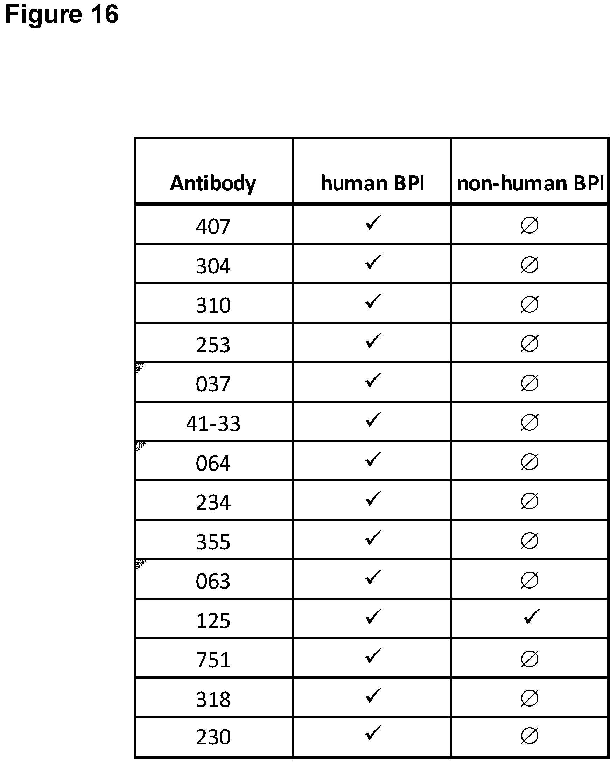

**Figure 16****. Binding of the antibodies to BPI and BPI fragments.** Table indicating binding of the antibodies to human BPI and non-human BPI which was tested using murine BPI. Binding was tested using Luminex technology by measuring binding of the indicated antibodies to beads coupled with the respective protein. The recombinantly expressed human BPI comprises amino acid 32 to 487 of human BPI (SEQ ID NO: 105), the non-human BPI corresponds to the recombinantly expressed murine BPI amino acids 28 to 483 (SEQ ID NO: 106). Abbreviations: ✔ binding , Ø no binding.

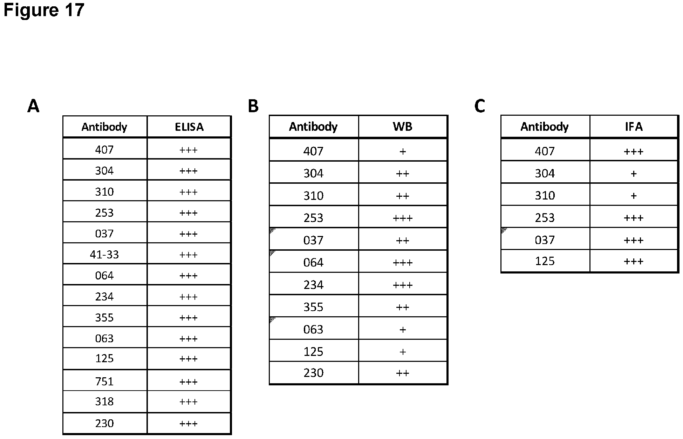

**Figure 17****. Diagnostic properties of the antibodies. (A)** Table indicating the diagnostic feasibility of the listed antibodies. For examples for a ELISA-related method see respective spider diagrams in Figures 6, 8, 9, 12, 13 and 14. **(B)** Table indicating the diagnostic feasibility of the listed antibodies for WB analysis. **(C)** Table indicating the diagnostic feasibility of the listed antibodies for IFA.
   Abbreviations: Western blot (WB), ImmunoFluorescence Assay (IFA), Enzyme-Linked Immunosorbent Assay (ELISA), +++ very strong performance, ++ strong performance, + good performance.
**Figure 18****. Exemplary antigen-binding peptides of the invention.** The CDR sequences and KDs of variants 407, 304, 310, 253, 037, 41-33, 064, 234, 355, 063, 125, 751, 318, and 230 are depicted. Variants 407, 304, 310, 253, 037, 41-33, 064, 234, 355, 063, 125, 751, 318, and 230 are exemplary antigen-binding peptides of the invention. In one embodiment, the antigen-binding peptide of the invention comprises the CDR sequences of any of the variants shown in Figure 18.
**Figure 19****. Exemplary antigen-binding peptides of the invention.** The VH and VL sequences of variants 407, 304, 310, 253, 037, 41-33, 064, 234, 355, 063, 125, 751, 318, and 230 are depicted. Variants 407, 304, 310, 253, 037, 41-33, 064, 234, 355, 063, 125, 751, 318, and 230 are exemplary antigen-binding peptides of the invention. In one embodiment, the antigen-binding peptide of the invention comprises the VH sequence and/or VL sequence of any of the variants shown in Figure 19.

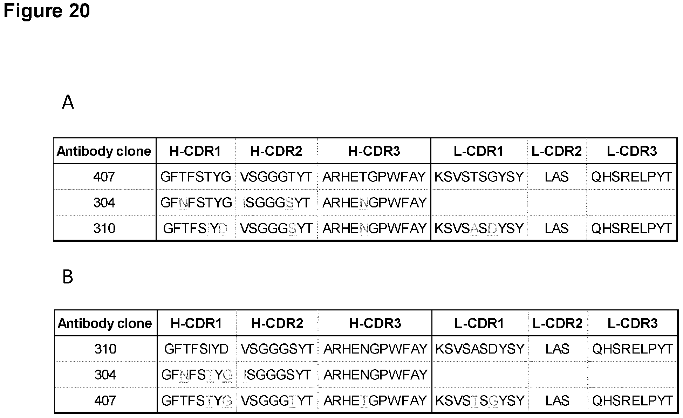

**Figure 20****. Sequence comparison of CDRs of different variants.** 4 amino acids are different in the heavy chain when comparing clone 304 to clone 407, and 4 amino acids are different in heavy chain and 2 amino acids are different in the light chain when comparing clone 310 to clone 407. Despite the differences in the sequences, the variants all inhibit the immunostimulatory activity of BPI. Amino acids changed in comparison to clone 407 are underlined (Figure 20 A). Amino acids changed in comparison to clone 310 are underlined (Figure 20 B). At least 8 amino acid positions do not seem to be critical with respect to the inhibitory capacity.

In the following, reference is made to the examples, which are given to illustrate, not to limit the present invention.

### EXAMPLES

### Example 1: Methods

### Cloning, production, and purification of recombinant BPI

To generate recombinant BPI, human BPI (aa 32-487) containing a C-terminal Flag was cloned into pCR3 vector (Thermo Fisher Scientific, Waltham, MA, USA). Expi293F cells were transfected using the ExpiFectamine 293 Transfection Kit (Thermo Fisher Scientific, Waltham, MA, USA). The expressed protein was purified by affinity chromatography on an αFlag (M2; Sigma Aldrich, Taufkirchen, Germany) coupled NHS-activated HP column (Cytiva, Marlborough, MA, USA), eluted with Flag-Peptide (peptides&elephants, Berlin, Germany), concentrated via ultrafiltration (Amicon Ultracel-PL, Merck Millipore, Darmstadt, Germany) and dialyzed against PBS. The concentration was determined by DC-Protein Assays (Bio-Rad Laboratories, Feldkirchen, Germany). In addition, indicated batches were purified by cation exchange chromatography via a HiTrap SP HP column (Cytiva, Marlborough, MA, USA). Fractions containing the protein of interest were pooled and further purified by size exclusion chromatography using a HighLoad 16/600Superdex 75 pg column (Cytiva, Marlborough, MA, USA).

### Generation and purification of BPI antibodies

Antibodies directed against human BPI were generated by immunization of male mice with recombinant BPI (generated as described above) by Davids Biotechnology (Regensburg, Germany) to generate hybridoma clones. These clones were screened by ELISA for production of antibodies directed against human BPI. Positive IgG clones were selected for expansion and antibodies were purified via HiTrap Protein An HP and HiTrap Protein G HP antibody purification columns (Cytiva Europe GmbH, Freiburg, Germany).

### Determination of the dissociation constant K_{D}

The dissociation constant was determined using an ELISA-based method whereby BPI was coated on plates and incubated with increasing concentrations of the respective monoclonal antibody. Affinity was calculated as the dissociation constant K_{D}.

### Cell culture

Cells were maintained in RPMI 1640 (Thermo Fisher Scientific, Waltham, MA, USA) supplemented with 10% heat-inactivated FCS (Sigma Aldrich, Taufkirchen, Germany), 10% penicillin, 10% streptomycin (both PAN-Biotech, Aidenbach, Germany) and 50 µM β-mercaptoethanol (AppliChem, Darmstadt, Germany). For BMDC generation, bone marrow cells three to four month old male C57BL/6J mice was plated in medium supplemented with supernatant of GM-CSF producing X63 cells. Fresh medium was added on days three and six. Loose as well as semi-adherent cells were harvested on day seven or eight. Generation of conventional type 1 DC from mouse bone marrow was performed using recombinant human FLT3L (Thermo Fisher Scientific, Waltham, MA, USA) and supernatant of GM-CSF producing X63 cells. For inhibition experiments, BMDCs were pre-incubated with indicated concentrations of neutralizing αBPI antibodies and consecutively stimulated with BPI.

### Quantification of cytokine levels

To analyze concentrations of cytokines in cell culture supernatant and serum, Luminex technology (Austin, TX, USA) was used. Briefly, antibodies to analyze IL-2 (capture αIL-2 JES6-1A12 and detection αIL-2 JES6-5H4), IL-6 (IL-6 ELISA Set), IL-10 (IL-10 ELISA Set), IL-12B (capture αIL-12p40/p70 C15.6 and detection αIL-12p40/p70 C17.8), CCL2 (capture αCCL2 2H5 and detection αCCL2 4E2/MCP) and TNF (TNF ELISA Set) were purchased from BD Bioscience (Heidelberg, Germany). IL-23 was detected using IL-23 ELISA Set (R&D Systems, Minneapolis, MN, USA). For detection Streptavidin-PE was used (Agilent, Palo Alto, CA, USA). Samples were measured with the Luminex 100 system and analyzed by using LiquiChip Analyzer Software (QIAGEN, Hilden, Germany).

### Example 2: Determination of epitopes

A peptide scan of the protein sequence of BPI with a peptide length of 15 amino acids and frame shift of 4 amino acids was purchased from peptides & elephants GmbH (Hennigsdorf, Germany). First, the membrane containing the peptide spots was incubated in methanol for 5 min, then washed 3 times with 0.05% TBS-T (Tris-Buffered Saline; 50mM Tris, 137mM NaCl, 2.7mM KCl, 0.05% Tween 20, pH 8.0) and blocked with 5% milk powder in TBS-T for 2 hours. The respective antibody was added in 5% milk powder in TBS-T. Bound antibody was detected with peroxidase-labeled antibodies rabbit anti-mouse (Dianova, Hamburg, Germany) and SuperSignal West Pico PLUS Chemiluminescent Substrate (Thermofisher Scientific, Waltham, MA, USA).

The identified epitopes are as follows:

| Variant | Epitope |
|---|---|
| 407 | FPAAHDRMVYLGLSD |
| 304 | FPAAHDRMVYLGLSD |
| 310 | FPAAHDRMVYLGLSD |
| 253 | FPAAHDRMVYLGLSD |
| 037 | FPAAHDRMVYLGLSD |
| 41-33 | VYLGLSDYFFNTAGLVYQE |
| 064 | PVMEFPAAHDRMVYLGLSD |
| 234 | PVMEFPAAHDRMVYLGLSD |
| 355 | PVMEFPAAHDRMVYLGLSD |
| 063 | HYSFYSMDIREFQLP, MVPNVGLKFSISNANIKIS, QKRFLKMSGNFDLSI |
| 125 | TFLPEVAKKFPNM |
| 230 | FQTLPVMTKIDSVAGINYLVAP |

### Example 3: Measurement of affinity

As described in example 1, the dissociation constant was determined using an ELISA-based method whereby BPI was coated on plates and incubated with increasing concentrations of the respective monoclonal antibody. Affinity was calculated as the dissociation constant K_{D} with results as follows:

| Antibody clone | Kd nM |
|---|---|
| 407 | 0.316 |
| 304 | 0.192 |
| 310 | 0.068 |
| 253 | 0.247 |
| 037 | 0.254 |
| 41-33 | 1.084 |
| 064 | 4.462 |
| 234 | 4.998 |
| 355 | 6.915 |
| 063 | 0.251 |
| 125 | 0.088 |
| 751 | 1.087 |
| 318 | 0.327 |
| 230 | 9.749 |

Affinity to BPI is particularly very high for antibodies 310 and 125 with a K_{D} of < 0.1 nM. Affinity for antibody 304, 253, 063, 037, 407 and 318 is also very high with a K_{D} of < 1.0 nM. Affinity for antibody 41-33, 751, 064, 234, 255 and 230 is high with a K_{D} of < 10 nM. Interestingly, Figure 5 shows the capacity to neutralize the immunostimulatory potential of BPI of the antigen-binding peptides of the invention, e.g. of variants 310, 125, 304, 253, 063, 037, 407, 318, 751 and 41-33.

### Example 4: Antigen-binding peptides of the invention inhibit the immunostimulatory activity of BPI

The inventors identified a novel, immunostimulatory function of human BPI in murine bone marrow-derived dendritic cells (BMDCs), a characteristic commonly attributed to endogenous immune activating proteins called alarmins, also termed danger associated molecular patterns (DAMPs, Figure 1). Moreover, as shown in Figure 2 endogenous danger signals such as TNF or GM-CSF can surprisingly trigger BPI-responsiveness. It is known that BPI is preformed in neutrophils. Release of BPI from neutrophils is triggered upon recognition of LPS or endogenous danger signals, such as TNF. Therefore, elevated levels of BPI are found both locally in inflammatory sites such as joints in RA or systemically such as plasma or serum in sepsis.

BPI is present in diseases where a high influx and/or activation of neutrophils is present such as in autoimmune diseases, e.g. rheumatoid arthritis, psoriasis or SLE, in inflammatory bowel diseases, and in bacterial infections such as sepsis. Increased neutrophil activation and/or neutrophil influx is also known for inflammaging, arthritis such as activated arthrosis, arteriosclerosis, tendinopathy, ischemia-perfusion-injury such as myocardial infarction, ischemic stroke or hemorrhagic stroke, complications of transplantation such as graft rejection or graft-versus-host-disease, and radiation induced tissue damage. Thus, tissue damage caused by infection, respiratory disease such as chronic obstructive lung disease or bronchiectasis, autoimmunity, ischemia, trauma, aberrant T cell activation, chemical toxicity or radiation can cause increased neutrophil influx and neutrophil activation. It is also known that eosinophils also contain preformed BPI. Therefore, allergy or parasitosis can also lead to increased BPI levels both locally and systemically. Malignancies of myeloid origin such as acute or chronic myeloid leukemia where leukemia cells produce BPI. Inflammation is associated with initiation and progression of myeloid neoplasms. All conditions mentioned in this example cause inflammation which can be detrimental und worsen disease outcome. Even in infection, where inflammation helps to control the infection, overwhelming inflammation like in sepsis can be fatal.

Since BPI drives inflammation (Figure 4), inhibition of BPI by a BPI-neutralizing antibody improves inflammation.

### Example 5: Antigen-binding peptides of the invention do not inhibit the antibiotic activity of BPI

Bactericidal/permeability-increasing protein (BPI) possesses a high affinity for the lipid A moiety of lipopolysaccharide (LPS), which originates from Gram-negative (GN) bacteria. The interaction with LPS is mediated by a cationic N-terminal barrel and disrupts the membrane integrity of GN bacteria. Additionally, BPI neutralizes the pro-inflammatory potential of LPS and facilitates phagocytosis of bound bacteria by the opsonizing function of its C-terminal domain. Due to selective bactericidal activity, anti-inflammatory capacity, and opsonizing properties, BPI holds therapeutic potential in GN infection including multi drug-resistant strains. Auto-antibodies directed against BPI commonly occur in people with cystic fibrosis. These antibodies can neutralize the bactericidal activity of BPI. Occurrence of auto-antibodies directed against BPI in people with cystic fibrosis is therefore associated with a decrease in pulmonary function and poor outcome. Ideally, an antigen-binding peptide binding to BPI does not inhibit the antibacterial activity of BPI, e.g. so that BPI may perform its antibacterial function and may prevent Gram-negative infections.

As shown in Figure 15, the antigen-binding peptides of the invention, e.g. variants 407, 310, 253, 037, 063, 125 and 751, are capable of inhibiting the inflammatory potential of BPI while surprisingly not significantly inhibiting the bactericidal activity of BPI. Therefore, these antibodies are especially suitable for application in a patient, such as in patients suffering from or being at risk of acquiring a bacterial infection such as an infection with Gram-negative bacteria.

The inventors have surprisingly found that the antigen-binding peptides of the invention, such as antigen-binding peptides binding to an epitope of BPI having a sequence of SEQ ID NO: 1, e.g. variants 407, 310, 253 and 037, are highly suitable for the treatment of patients. For example, the epitope of BPI having a sequence of SEQ ID NO: 1 is highly suitable for generating antigen-binding peptides or small molecules for a therapy of a patient. Furthermore, the inventors have found that, for example, the epitope defined by SEQ ID NO: 4 (e.g. bound by variant 125) as well as the epitope defined by SEQ ID NOs: 102, 103 and 104 (e.g. bound by variants 063 and 751) are highly suitable for generating antigen-binding peptides or small molecules for a therapy of a patient.

Thus, surprisingly, the epitopes defined herein, such as epitopes as defined by SEQ ID NO: 1, SEQ ID NO: 4 and SEQ ID NO: 102, 103 and 104, are of particular interest when generating antigen-binding peptides or small molecules for a therapy of a patient.

### Example 6: Roadmap for identifying agents binding to the epitopes defined herein, such as antigen-binding peptides or small molecules

The method of identifying an agent binding to BPI, particularly to generate agents inhibiting the immunostimulatory potential of BPI, may comprise generating a library of agents, such as candidate antibodies or antigen-binding fragments thereof, or candidate small molecules, and then test them to determine whether they bind to any of the epitopes defined herein, particularly any of the sweet spots (such as the epitopes as defined by SEQ ID NO: 1, SEQ ID NO: 4 and SEQ ID NO: 102-104), and optionally further determining whether they inhibit the immunostimulatory potential of BPI, e.g. by measuring an IL-2 level after stimulation of BPI-responsive cells. For example, if the agent to be tested is an antibody, the entire BPI molecule may be used for immunization and then a screening for inhibitory antibodies that inhibit the immunostimulatory potential of BPI but do not inhibit bactericidal activity may be performed. Alternatively, the epitope sequences may be used for immunization instead of the entire BPI molecule, preferably sequences defining the sweet spots, more preferably a sequence of SEQ ID NO:1. For example, if the agent to be tested is a small molecule, the defined epitopes may be used for screening small molecules binding to said epitopes, preferably sequences defining the sweet spots, more preferably of SEQ ID NO:1.

**Example 6:** Method of identifying an agent binding to BPI, e.g. Conservative Substitution The method of identifying an agent binding to BPI may comprise using a known antigen-binding peptide of the genus (e.g., one of the variants described herein), replace selected amino acids, and then test the resulting antigen-binding peptides to see if they are effective inhibitors of the immunostimulatory potential of BPI, preferably if they inhibit the immunostimulatory potential of BPI without inhibiting the bactericidal activity of BPI. For example, selected amino acids may be replaced in the CDRs binding to any of the indicated epitopes, particularly in the sweet spots, for example to an epitope having SEQ ID NO: 1, and the resulting peptide may be tested with respect to whether it inhibits the immunostimulatory activity of BPI, preferably whether it inhibits the immunostimulatory activity of BPI but does not inhibit the bactericidal activity of BPI.

For example, the inventors have found with respect to antigen-binding peptides binding to an epitope having SEQ ID NO: 1, that, although 4 amino acids are different in the heavy chain when comparing clone 304 to clone 407, and 4 amino acids are different in heavy chain and 2 amino acids are different in the light chain when comparing clone 310 to clone 407, the inhibitory capacity is still preserved. With an exchange of few amino acids, such as one amino acid, the probability is high that an inhibition of the immunostimulatory effect of BPI is obtained when using antigen-binding peptides such as antibodies that recognize an epitope having SEQ ID NO: 1. Amino acids changed in comparison to clone 407 are underlined (Figure 20 A). Thus, a total of at least 8 amino acid positions within the CDR of antibody 407 do not seem to be critical with respect to the inhibitory capacity. Since 8 amino acids of the 49 amino acids of the CDR of antibody 407 compromise 83.67% of the CDR sequence of antibody 407, an exchange of 16.33% of the amino acids of the CDR of an antibody does not seem to critically influence the function of antibody 407.

For example, the inventors have found with respect to antigen-binding peptides binding to an epitope having SEQ ID NO: 1, that, although 4 amino acids are different in the heavy chain when comparing clone 304 to clone 310, and 4 amino acids are different in heavy chain and 2 amino acids are different in the light chain when comparing clone 407 to clone 310, the inhibitory capacity is still preserved. With an exchange of few amino acids, such as one amino acid, the probability is high that an inhibition of the immunostimulatory effect of BPI is obtained when using antigen-binding peptides such as antibodies that recognize an epitope having SEQ ID NO: 1. Amino acids changed in comparison to clone 310 are underlined (Figure 20 B). Thus, a total of at least 8 amino acid positions within the CDR of antibody 310 do not seem to be critical with respect to the inhibitory capacity. Since 8 amino acids of the 49 amino acids of the CDR of antibody 310 compromise 83.67% of the CDR sequence of antibody 310, an exchange of 16.33% of the amino acids of the CDR of an antibody does not seem to critically influence the function of antibody 310.

The features of the present invention disclosed in the specification, the claims, and/or in the accompanying figures may, both separately and in any combination thereof, be material for realizing the invention in various forms thereof.

## Claims

1. An antigen-binding peptide binding to bactericidal permeability-increasing protein (BPI), wherein said antigen-binding peptide binds to an epitope of BPI comprising or consisting of an amino acid sequence selected from
| | |
|---|---|
| FPAAHDRMVYLGLSD | SEQ ID NO: 1; |
| VYLGLSDYFFNTAGLVYQE | SEQ ID NO: 2; |
| PVMEFPAAHDRMVYLGLSD | SEQ ID NO: 3; |
| TFLPEVAKKFPNM | SEQ ID NO: 4; |
| FQTLPVMTKIDSVAGINYLVAP | SEQ ID NO: 5; |
| HYSFYSMDIREFQLP | SEQ ID NO: 102; |
| MVPNVGLKFSISNANIKIS | SEQ ID NO: 103; and |
| QKRFLKMSGNFDLSI | SEQ ID NO: 104. |

2. The antigen-binding peptide according to claim 1, comprising any one of a)-n):
a) a heavy chain complementarity-determining region (HCDR) 1 comprising the amino acid sequence GFNFSTYG [SEQ ID NO: 6],
a HCDR2 comprising the amino acid sequence ISGGGSYT [SEQ ID NO: 7], and
a HCDR3 comprising the amino acid sequence ARHENGPWFAY [SEQ ID NO: 8]; or
b)
a HCDR 1 comprising the amino acid sequence GFTFSIYD [SEQ ID NO: 9],
a HCDR2 comprising the amino acid sequence VSGGGSYT [SEQ ID NO: 10],
a HCDR3 comprising the amino acid sequence ARHENGPWFAY [SEQ ID NO: 11],
a LCDR 1 comprising the amino acid sequence KSVSASDYSY [SEQ ID NO: 12],
a LCDR2 comprising the amino acid sequence LAS [SEQ ID NO: 13], and
a LCDR3 comprising the amino acid sequence QHSRELPYT [SEQ ID NO: 14]; or
c)
a HCDR 1 comprising the amino acid sequence GFTFSTYG [SEQ ID NO: 15],
a HCDR2 comprising the amino acid sequence VSGGGTYT [SEQ ID NO: 16],
a HCDR3 comprising the amino acid sequence ARHETGPWFAY [SEQ ID NO: 17],
a LCDR 1 comprising the amino acid sequence KSVSTSGYSY [SEQ ID NO: 18],
a LCDR2 comprising the amino acid sequence LAS [SEQ ID NO: 19], and
a LCDR3 comprising the amino acid sequence QHSRELPYT [SEQ ID NO: 20]; or
d)
a HCDR 1 comprising the amino acid sequence GFSLISSA [SEQ ID NO: 21],
a HCDR2 comprising the amino acid sequence IWAGGST [SEQ ID NO: 22],
a HCDR3 comprising the amino acid sequence ATYYRFDDGFAY [SEQ ID NO: 23],
a LCDR 1 comprising the amino acid sequence QSLVHSSGITY [SEQ ID NO: 24],
a LCDR2 comprising the amino acid sequence RVS [SEQ ID NO: 25], and
a LCDR3 comprising the amino acid sequence SQSTHFPYT [SEQ ID NO: 26]; or
e)
a HCDR 1 comprising the amino acid sequence GFTFSNYG [SEQ ID NO: 27],
a HCDR2 comprising the amino acid sequence ISGGGNYT [SEQ ID NO: 28],
a HCDR3 comprising the amino acid sequence ARREAQLGSAMDY [SEQ ID NO: 29],
a LCDR 1 comprising the amino acid sequence KSVSTSGYSY [SEQ ID NO: 30],
a LCDR2 comprising the amino acid sequence LAS [SEQ ID NO: 31], and
a LCDR3 comprising the amino acid sequence QHSRELPLT [SEQ ID NO: 32]; or
f)
a HCDR 1 comprising the amino acid sequence GYEFSISW [SEQ ID NO: 33],
a HCDR2 comprising the amino acid sequence IFPGDGDT [SEQ ID NO: 341,
a HCDR3 comprising the amino acid sequence AREVGR [SEQ ID NO: 35],
a LCDR 1 comprising the amino acid sequence QDIYNY [SEQ ID NO: 36],
a LCDR2 comprising the amino acid sequence YTS [SEQ ID NO: 37], and
a LCDR3 comprising the amino acid sequence QQGNTLPYT [SEQ ID NO: 38]; or
g)
a LCDR 1 comprising the amino acid sequence QSIGTS [SEQ ID NO: 39],
a LCDR2 comprising the amino acid sequence YAS [SEQ ID NO: 40], and
a LCDR3 comprising the amino acid sequence QQSNSWPLT [SEQ ID NO: 41]; or
h)
a HCDR 1 comprising the amino acid sequence GFDFDRYW [SEQ ID NO: 42],
a HCDR2 comprising the amino acid sequence INPNNNTI [SEQ ID NO: 43],
a HCDR3 comprising the amino acid sequence ARPGFYYGSAWFAY [SEQ ID NO: 44],
a LCDR 1 comprising the amino acid sequence TGAVTTSNY [SEQ ID NO: 45],
a LCDR2 comprising the amino acid sequence GSN [SEQ ID NO: 46], and
a LCDR3 comprising the amino acid sequence ALWYNSHWV [SEQ ID NO: 47]; or
i)
a HCDR 1 comprising the amino acid sequence GYTFTSYP [SEQ ID NO: 48],
a HCDR2 comprising the amino acid sequence FHPYNDDT [SEQ ID NO: 49],
a HCDR3 comprising the amino acid sequence ARAFYGNYDY [SEQ ID NO: 50],
a LCDR 1 comprising the amino acid sequence QSVNND [SEQ ID NO: 51],
a LCDR2 comprising the amino acid sequence YAS [SEQ ID NO: 52], and
a LCDR3 comprising the amino acid sequence HQNNRSPWT [SEQ ID NO: 53]; or
j)
a LCDR 1 comprising the amino acid sequence DHINNW [SEQ ID NO: 54],
a LCDR2 comprising the amino acid sequence GTT [SEQ ID NO: 55], and
a LCDR3 comprising the amino acid sequence QQYWSSPYT [SEQ ID NO: 56]; or
k)
a HCDR 1 comprising the amino acid sequence GFTFSNYW [SEQ ID NO: 57],
a HCDR2 comprising the amino acid sequence IRLKSNNYAT [SEQ ID NO: 58],
a HCDR3 comprising the amino acid sequence TQSYYSMDY [SEQ ID NO: 59],
a LCDR 1 comprising the amino acid sequence QSLLNSGNQKNY [SEQ ID NO: 60],
a LCDR2 comprising the amino acid sequence GAS [SEQ ID NO: 61], and
a LCDR3 comprising the amino acid sequence QNDHSYPRT [SEQ ID NO: 62]; or
l)
a HCDR 1 comprising the amino acid sequence GFTFSNYW [SEQ ID NO: 63],
a HCDR2 comprising the amino acid sequence IRLKSNNYAT [SEQ ID NO: 64],
a HCDR3 comprising the amino acid sequence TQSYYSMDY [SEQ ID NO: 65],
a LCDR 1 comprising the amino acid sequence QSLLYSSNQKNF [SEQ ID NO: 66],
a LCDR2 comprising the amino acid sequence WAS [SEQ ID NO: 67], and
a LCDR3 comprising the amino acid sequence QQYYNYPYT [SEQ ID NO: 68]; or
m)
a HCDR 1 comprising the amino acid sequence GFTFSNYW [SEQ ID NO: 69],
a HCDR2 comprising the amino acid sequence IRLRSNNYAS [SEQ ID NO: 70], and
a HCDR3 comprising the amino acid sequence AQSYYAMDY [SEQ ID NO: 71]; or
n)
a HCDR 1 comprising the amino acid sequence GFNIKDSY [SEQ ID NO: 72],
a HCDR2 comprising the amino acid sequence IDPANGDT [SEQ ID NO: 73],
a HCDR3 comprising the amino acid sequence ALYGYRAY [SEQ ID NO: 74],
a LCDR 1 comprising the amino acid sequence QSLLNSGNQKNY [SEQ ID NO: 75],
a LCDR2 comprising the amino acid sequence GAS [SEQ ID NO: 76], and
a LCDR3 comprising the amino acid sequence QNDHSYPRT [SEQ ID NO: 77];
wherein, preferably, the antigen-binding peptide comprises any one of a)-j);
wherein, more preferably, the antigen-binding peptide comprises any one of b)-f), i) and h);
wherein, even more preferably, the antigen-binding peptide comprises any one of b)-f) and i).

3. The antigen-binding peptide according to claim 1 or 2, wherein the antigen-binding peptide comprises a heavy chain variable sequence (VH) and a light chain variable sequence (VL), wherein VH and VL have at least 80%, preferably at least 90%, more preferably at least 95%, even more preferably at least 99%, optionally 100%, sequence identity to the amino acid sequences of:
| | | |
|---|---|---|
| i) | VH | SEQ ID NO: 78; or |
| ii) | VH | SEQ ID NO: 79 and |
| | VL | SEQ ID NO: 80, respectively; or |
| iii) | VH | SEQ ID NO: 81 and |
| | VL | SEQ ID NO: 82, respectively; or |
| iv) | VH | SEQ ID NO: 83 and |
| | VL | SEQ ID NO: 84, respectively; or |
| v) | VH | SEQ ID NO: 85 and |
| | VL | SEQ ID NO: 86, respectively; or |
| vi) | VH | SEQ ID NO: 87 and |
| | VL | SEQ ID NO: 88, respectively; or |
| vii) | VL | SEQ ID NO: 89, respectively; or |
| viii) | VH | SEQ ID NO: 90 and |
| | VL | SEQ ID NO: 91, respectively; or |
| ix) | VH | SEQ ID NO: 92 and |
| | VL | SEQ ID NO: 93, respectively; or |
| x) | VL | SEQ ID NO: 94, respectively; or |
| xi) | VH | SEQ ID NO: 95 and |
| | VL | SEQ ID NO: 96, respectively; or |
| xii) | VH | SEQ ID NO: 97 and |
| | VL | SEQ ID NO: 98, respectively; or |
| xiii) | VH | SEQ ID NO: 99; or |
| xiv) | VH | SEQ ID NO: 100 and |
| | VL | SEQ ID NO: 101, respectively; |
wherein, preferably, the antigen-binding peptide comprises heavy and light chain variable sequences having at least 80%, preferably at least 90%, more preferably at least 95%, optionally identity, to the amino acid sequences of any one of i) - x);
wherein, more preferably, the antigen-binding peptide comprises heavy and light chain variable sequences having at least 80%, preferably at least 90%, more preferably at least 95%, optionally identity, to the amino acid sequences of any one of ii) - vi) and viii) - ix);
wherein, even more preferably, the antigen-binding peptide comprises heavy and light chain variable sequences having at least 80%, preferably at least 90%, more preferably at least 95%, optionally identity, to the amino acid sequences of any one of ii) - vi) and ix).

4. The antigen-binding peptide according to any one of the foregoing claims, wherein said antigen-binding peptide binds to an epitope having SEQ ID NO: 1 and comprises
a) a heavy chain complementarity-determining region (HCDR) 1 comprising the amino acid sequence GFNFSTYG [SEQ ID NO: 6],
a HCDR2 comprising the amino acid sequence ISGGGSYT [SEQ ID NO: 7], and
a HCDR3 comprising the amino acid sequence ARHENGPWFAY [SEQ ID NO: 8];
or
b)
a HCDR 1 comprising the amino acid sequence GFTFSIYD [SEQ ID NO: 9],
a HCDR2 comprising the amino acid sequence VSGGGSYT [SEQ ID NO: 10],
a HCDR3 comprising the amino acid sequence ARHENGPWFAY [SEQ ID NO: 11],
a LCDR 1 comprising the amino acid sequence KSVSASDYSY [SEQ ID NO: 12],
a LCDR2 comprising the amino acid sequence LAS [SEQ ID NO: 13], and
a LCDR3 comprising the amino acid sequence QHSRELPYT [SEQ ID NO: 14]; or
c)
a HCDR 1 comprising the amino acid sequence GFTFSTYG [SEQ ID NO: 15],
a HCDR2 comprising the amino acid sequence VSGGGTYT [SEQ ID NO: 16],
a HCDR3 comprising the amino acid sequence ARHETGPWFAY [SEQ ID NO: 17],
a LCDR 1 comprising the amino acid sequence KSVSTSGYSY [SEQ ID NO: 18],
a LCDR2 comprising the amino acid sequence LAS [SEQ ID NO: 19], and
a LCDR3 comprising the amino acid sequence QHSRELPYT [SEQ ID NO: 20]; or
d)
a HCDR 1 comprising the amino acid sequence GFSLISSA [SEQ ID NO: 21],
a HCDR2 comprising the amino acid sequence IWAGGST [SEQ ID NO: 22],
a HCDR3 comprising the amino acid sequence ATYYRFDDGFAY [SEQ ID NO: 23],
a LCDR 1 comprising the amino acid sequence QSLVHSSGITY [SEQ ID NO: 24],
a LCDR2 comprising the amino acid sequence RVS [SEQ ID NO: 25], and
a LCDR3 comprising the amino acid sequence SQSTHFPYT [SEQ ID NO: 26]; or
e)
a HCDR 1 comprising the amino acid sequence GFTFSNYG [SEQ ID NO: 27],
a HCDR2 comprising the amino acid sequence ISGGGNYT [SEQ ID NO: 28],
a HCDR3 comprising the amino acid sequence ARREAQLGSAMDY [SEQ ID NO: 29],
a LCDR 1 comprising the amino acid sequence KSVSTSGYSY [SEQ ID NO: 30],
a LCDR2 comprising the amino acid sequence LAS [SEQ ID NO: 31], and
a LCDR3 comprising the amino acid sequence QHSRELPLT [SEQ ID NO: 32];
or wherein said antigen-binding peptide binds to an epitope having SEQ ID NO: 2 and comprises
g)
a LCDR 1 comprising the amino acid sequence QSIGTS [SEQ ID NO: 39],
a LCDR2 comprising the amino acid sequence YAS [SEQ ID NO: 40], and
a LCDR3 comprising the amino acid sequence QQSNSWPLT [SEQ ID NO: 41];
or wherein said antigen-binding peptide binds to an epitope having SEQ ID NO: 3 and comprises
k)
a HCDR 1 comprising the amino acid sequence GFTFSNYW [SEQ ID NO: 57],
a HCDR2 comprising the amino acid sequence IRLKSNNYAT [SEQ ID NO: 58],
a HCDR3 comprising the amino acid sequence TQSYYSMDY [SEQ ID NO: 59],
a LCDR 1 comprising the amino acid sequence QSLLNSGNQKNY [SEQ ID NO: 60],
a LCDR2 comprising the amino acid sequence GAS [SEQ ID NO: 61], and
a LCDR3 comprising the amino acid sequence QNDHSYPRT [SEQ ID NO: 62]; or
l)
a HCDR 1 comprising the amino acid sequence GFTFSNYW [SEQ ID NO: 63],
a HCDR2 comprising the amino acid sequence IRLKSNNYAT [SEQ ID NO: 64],
a HCDR3 comprising the amino acid sequence TQSYYSMDY [SEQ ID NO: 65],
a LCDR 1 comprising the amino acid sequence QSLLYSSNQKNF [SEQ ID NO: 66],
a LCDR2 comprising the amino acid sequence WAS [SEQ ID NO: 67], and
a LCDR3 comprising the amino acid sequence QQYYNYPYT [SEQ ID NO: 68]; or
m)
a HCDR 1 comprising the amino acid sequence GFTFSNYW [SEQ ID NO: 69],
a HCDR2 comprising the amino acid sequence IRLRSNNYAS [SEQ ID NO: 70], and
a HCDR3 comprising the amino acid sequence AQSYYAMDY [SEQ ID NO: 71];
or wherein said antigen-binding peptide binds to an epitope having SEQ ID NO: 4 and comprises
f)
a HCDR 1 comprising the amino acid sequence GYEFSISW [SEQ ID NO: 33],
a HCDR2 comprising the amino acid sequence IFPGDGDT [SEQ ID NO: 341,
a HCDR3 comprising the amino acid sequence AREVGR [SEQ ID NO: 35],
a LCDR 1 comprising the amino acid sequence QDIYNY [SEQ ID NO: 36],
a LCDR2 comprising the amino acid sequence YTS [SEQ ID NO: 37], and
a LCDR3 comprising the amino acid sequence QQGNTLPYT [SEQ ID NO: 38];
or wherein said antigen-binding peptide binds to an epitope having SEQ ID NO: 5 and comprises
n)
a HCDR 1 comprising the amino acid sequence GFNIKDSY [SEQ ID NO: 72],
a HCDR2 comprising the amino acid sequence IDPANGDT [SEQ ID NO: 73],
a HCDR3 comprising the amino acid sequence ALYGYRAY [SEQ ID NO: 74],
a LCDR 1 comprising the amino acid sequence QSLLNSGNQKNY [SEQ ID NO: 75],
a LCDR2 comprising the amino acid sequence GAS [SEQ ID NO: 76], and
a LCDR3 comprising the amino acid sequence QNDHSYPRT [SEQ ID NO: 77];
or wherein said antigen-binding peptide binds to an epitope having any one of SEQ ID NO: 102-104 and comprises
i)
a HCDR 1 comprising the amino acid sequence GYTFTSYP [SEQ ID NO: 48],
a HCDR2 comprising the amino acid sequence FHPYNDDT [SEQ ID NO: 49],
a HCDR3 comprising the amino acid sequence ARAFYGNYDY [SEQ ID NO: 50],
a LCDR 1 comprising the amino acid sequence QSVNND [SEQ ID NO: 51],
a LCDR2 comprising the amino acid sequence YAS [SEQ ID NO: 52], and
a LCDR3 comprising the amino acid sequence HQNNRSPWT [SEQ ID NO: 53].

5. The antigen-binding peptide according to any one of the foregoing claims, wherein said antigen-binding peptide is an antibody or an antigen-binding fragment thereof; wherein, preferably, said antigen-binding peptide is selected from an antibody, a Fab, Fab', a F(ab')2, a scFv, di-scFv, a V_{H} domain, a single-domain antibody (sdAb), a diabody, a triabody, and a tetrabody;
wherein, more preferably, the antigen-binding peptide is selected from a humanized antibody, a chimeric antibody, and antigen-binding fragments thereof.

6. The antigen-binding peptide according to any one of the foregoing claims, wherein said antigen-binding peptide is an IgG2a antibody, an IgG2b antibody, an IgG1 antibody, or an antigen-binding fragment of an IgG2a antibody, of an IgG2b antibody, or of an IgG1 antibody;
wherein, optionally, said antigen binding peptide is selected from a humanized IgG2a antibody, a humanized IgG2b antibody, a humanized IgG1 antibody, and antigen-binding fragments thereof.

7. The antigen-binding peptide according to any one of the foregoing claims, wherein said antigen-binding peptide neutralizes said BPI;
wherein, preferably, said antigen-binding peptide inhibits a pro-inflammatory activity of BPI in immune cells, particularly dendritic cells, leukocytes, and/or macrophages; wherein, more preferably, said antigen-binding peptide inhibits an induction of cytokines, particularly IL-2 and/or TNF, by BPI in immune cells, particularly in dendritic cells, leukocytes, and/or macrophages, such as in bone marrow-derived dendritic cells.

8. The antigen-binding peptide according to any one of the foregoing claims, wherein said antigen-binding peptide does not inhibit an antibiotic activity of BPI, particularly a bactericidal activity of BPI.

9. The antigen-binding peptide according to any one of the foregoing claims, wherein said antigen-binding peptide has a KD of 10 nM or less, preferably has a KD of 7 nM or less, more preferably has a KD of 1.5 nM or less, even more preferably has a KD of 1.1 nM or less, still even more preferably has a KD of 0.5 nM or less, still even more preferably has a KD of 0.3 nM or less, such as a KD of 0.01 nM or less.

10. A nucleic acid, particularly an isolated nucleic acid, encoding an antigen-binding peptide according to any one of claims 1-9.

11. A recombinant cell comprising a nucleic acid according to claim 10.

12. A composition, preferably a pharmaceutical composition, comprising an antigen-binding peptide according to any one of claims 1-9, a nucleic acid according to claim 10, or a recombinant cell according to claim 11, and a pharmaceutically acceptable excipient.

13. An antigen-binding peptide according to any one of claims 1-9, a nucleic acid according to claim 10, a recombinant cell according to claim 11, or a composition according to claim 12, for use in a method of preventing or treating an inflammatory disease, disorder, or condition;
wherein, optionally, said inflammatory disease, disorder, or condition is selected from an infection, such as a sepsis; an autoimmune disease, such as rheumatoid arthritis, systemic lupus erythematosus, multiple sclerosis, psoriasis, and inflammatory bowel disease, e.g. Crohn's disease and ulcerative colitis; a neurodegenerative disease; an allergy; an inflammatory medical condition after a transplantation, such as a graft rejection and a graft-versus-host disease (GvHD); a respiratory disease, such as asthma and chronic obstructive pulmonary disease; inflammaging; arthritis, such as activated arthrosis; arteriosclerosis; tendinopathy; an ischemia-perfusion-injury, such as myocardial infarction, ischemic stroke, and hemorrhagic stroke; radiation induced tissue damage; and cancer, such as acute or chronic myeloid leukemia;
wherein, optionally, an anti-inflammatory agent other than the antigen-binding peptide, nucleic acid, recombinant cell, or composition is coadministered with said antigen-binding peptide, nucleic acid, recombinant cell, or composition, preferably selected from nonsteroidal anti-inflammatory drugs, corticosteroids such as cortisone, disease-modifying anti-rheumatic drugs, and antileukotriens, more preferably is coadministered with cortisone.

14. Use of an antigen-binding peptide according to any one of claims 1-9, a nucleic acid according to claim 10, a recombinant cell according to claim 11, or a composition according to claim 12, in a method of detecting BPI in a sample,
wherein, optionally, said method comprises detecting BPI in said sample using an enzyme-linked immunosorbent assay, an immunochromatography, a western blot, and/or an immunohistochemistry;
wherein, preferably, said use is an in vitro use and/or ex vivo use.

15. A method of identifying an agent binding to BPI, preferably an antigen-binding peptide binding to BPI or a small molecule binding to BPI, particularly specifically binding to BPI, comprising
a) providing at least one agent to be tested for a capability of binding, particularly specifically binding, to BPI;
b) testing whether said at least one agent binds to, particularly specifically binds to, an epitope of BPI comprising or consisting of an amino acid sequence selected from
| | |
|---|---|
| FPAAHDRMVYLGLSD | SEQ ID NO: 1; |
| VYLGLSDYFFNTAGLVYQE | SEQ ID NO: 2; |
| PVMEFPAAHDRMVYLGLSD | SEQ ID NO: 3; |
| TFLPEVAKKFPNM | SEQ ID NO: 4; |
| FQTLPVMTKIDSVAGINYLVAP | SEQ ID NO: 5; |
| HYSFYSMDIREFQLP | SEQ ID NO: 102; |
| MVPNVGLKFSISNANIKIS | SEQ ID NO: 103; and |
| QKRFLKMSGNFDLSI | SEQ ID NO: 104. |
